# EUROPEAN PATENT APPLICATION

(11) **EP 2 289 904 A1**
(43) Date of publication of application: **02.03.2011**
(21) Application number: 09380127.2
(22) Date of filing: 03.07.2009
(51) Int. Cl.: C07H 15/207, A61K 31/7034, A61P 31/12

(54) **Inhibitors of microbial infections**

(71) Applicant: Universita' degli Studi di Milano, 20122 Milano (IT); Consejo Superior De Investigaciones Científicas, 28006 Madrid (ES); Université Joseph Fourier, 38041 Grenoble Cedex 09 (FR); Centre National de la Recherche Scientifique, 75794 Paris Cedex 16 (FR)
(72) Inventor: Bernardi, Anna, 20133 Milano (IT); Sattin, Sara, 24020 Cene Bergamo (IT); Clerici, Mario Salvatore, 20129 Milano (IT); Berzi, Angela Maria Vittoria, 20133 Milano (IT); Rojo, Francisco Javier, 41927 Sevilla (ES); Sanchez, Macarena, 18004 Granada (ES); Reina, Joseph Juan, 29006 Malaga (ES); Fieschi, Franck, 38450 Vif (FR)
(74) Representative: Long, Giorgio

(57) **Abstract**

The present invention finds application in the field of medicine and, in particular, it relates to new compounds for the treatment and/or prevention of HIV; Ebola, Dengue, Hepatitis C, SARS or tuberculosis infections.

## Description

The present invention finds application in the field of medicine and, in particular, it relates to new inhibitors of microbial infections.

### BACKGROUND

Recognition of highly-glycosylated structures displayed at the surface of several pathogens such as viruses, bacteria, yeasts and parasites appears to play a key role in the infection process of many pathogens and it is considered to be an interesting new target for the design of anti-infective agents. At least for some pathogens, including Human Immunodeficiency Virus (HIV), Dengue virus, Ebola virus, Hepatitis virus, SARS and Mycobacterium tuberculosis, the interaction of highly mannosylated glycans with specific lectin receptors in immune system is an initial and essential step allowing the penetration of the pathogen within the host cells. By inhibiting glycans-mediated pathogen recognition, thus the initial stages of the infection could be prevented. The anti-adhesive mechanism by which such antagonists would work interferes with the pathogenic action of the pathogenic microorganisms, without killing it. Therapies based on anti-microbial drugs which kill pathogens apply a strong selective pressure to the microbial population, which in turns favours the emergence of resistant strains: hence the never-ending search for new, more powerful agents capable of fighting such strains. For viruses, this is particularly evident as a consequence of the rapid rate of mutations of viral population. The approach using anti-adhesive molecules would prevent infection while avoiding pathogen selective mechanisms, thus simply making the pathogen non harmful.

It has recently been reported that the pseudo-disaccharide 1 (see below), mimicking the three-dimensional structure and conformational behavior of a mannose disaccharide (Mana1-2Man), binds to one of the immune system receptors mentioned above, the dendritic cell receptor DC-SIGN, and exhibits moderate anti-infective action in a Ebola virus infection model (Reina J.J. et al. ChemMedChem, 2007, 2, 1030 - 1036). Multivalent, polymannosylated compounds were shown to bind to the same receptor and to inhibit its interaction to gp120-coated SPR chips (Rojo et al FEBS Letters 2006, 580, 2402-2408).

The pseudo-trisaccharide **2a** below (R=allyl) was recently designed as a mimic of the linear mannotrioside α-Man-(1-2)-a-Man-(1-6)-a-Man- by replacing the central mannose moiety with a carbocyclic diol (Mari, S.; Sánchez-Medina, I.; Mereghetti, P.; Belvisi, L.; Jiménez-Barbero, J.; Bernardi, A. Carb. Res. 2007, 342, 1859- 1868).

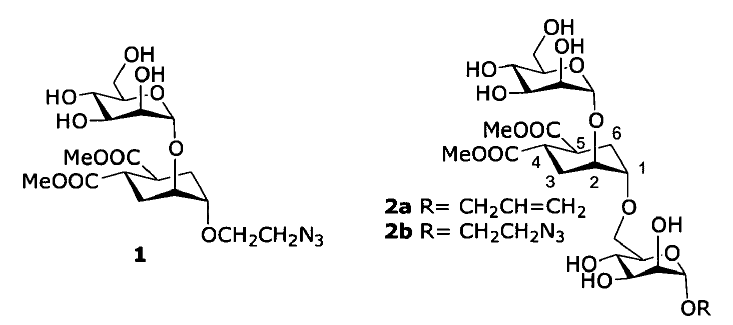

It is the general consensus that the development of efficient, low-cost microbicides and entry inhibitor molecules that can be applied topically to prevent sexually transmitted HIV infections should be given high priority. Mannan is known to inhibit viral uptake in cellular studies (Lin G. et al J. Virol. 2003, 77, 1337-46) but it cannot be used in vivo because it does not cross mucosal tissues and, as a consequence, it is unlikely to reach its target. Mannan is also highly cytotoxic and mitogenic, which limits its therapeutic applications in vivo (Nagase, T. et al. Microbiol. Immunol., 1984, 28, 997-1007).

Therefore, there is a need in the field of medicine for new compounds which are effective in inhibiting infections mediated by immune system lectins, including DC-SIGN which would be active as well as scarcely cytotoxic.

### SUMMARY OF THE INVENTION

The present invention, accordingly, provides new inhibitors of infections, which do not favour the emergence of resistant strains, has lower toxicity, may be applied topically and could cross mucoses significantly better compared to the already known compounds.

### OBJECT OF THE INVENTION

In a first object, the present invention discloses the compounds having inhibition activity of claims 1 to 12.

In a second object, the present invention concerns a process for the preparation of the compounds of the invention.

It is a third object the use of the compounds of the invention as a medicament for the treatment and/or prevention of HIV; Ebola, Dengue, Hepatitis C, SARS or tuberculosis infections as per claims 13 to 17.

It is a forth object of the invention a pharmaceutical preparation comprising one or more of the compounds of the invention as per claims 18 to 20.

### DESCRIPTION OF THE FIGURES

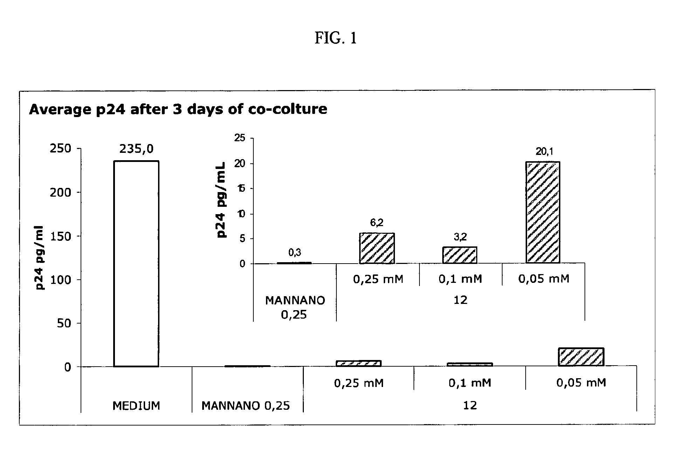

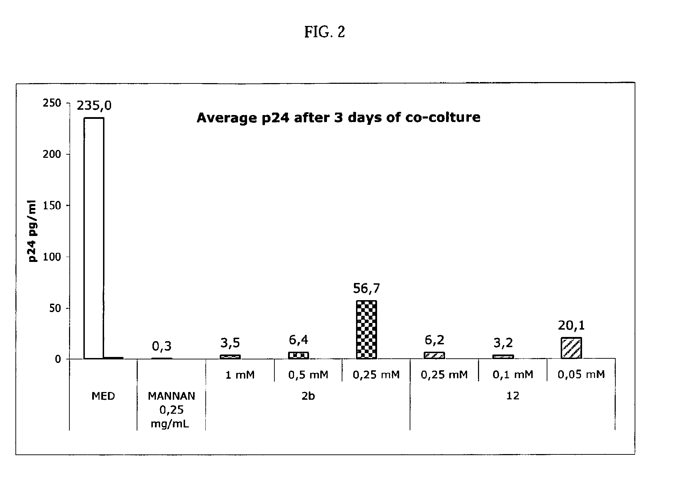

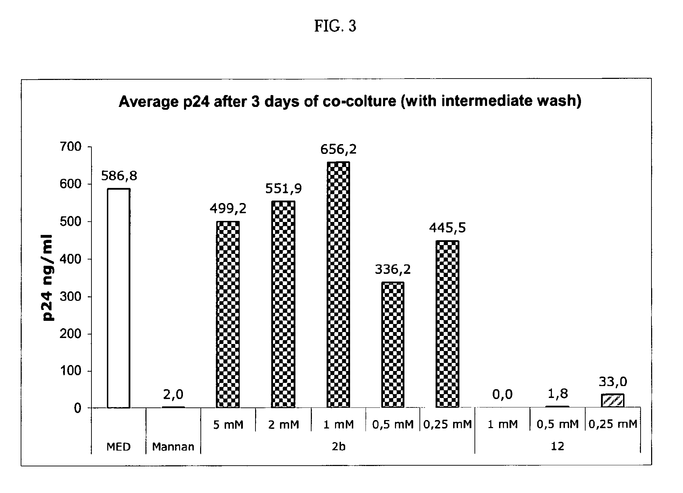

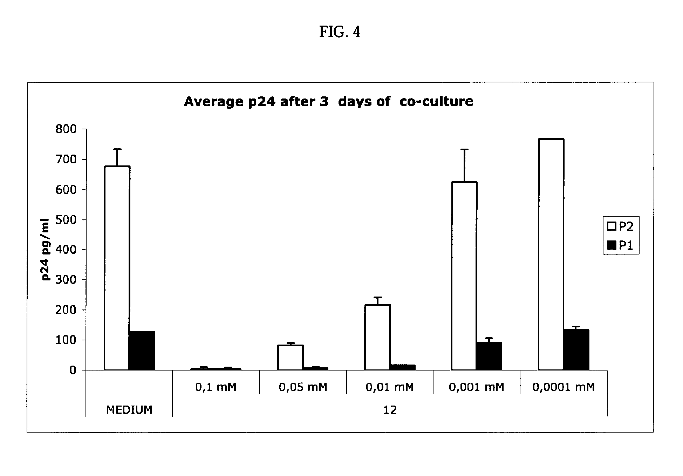

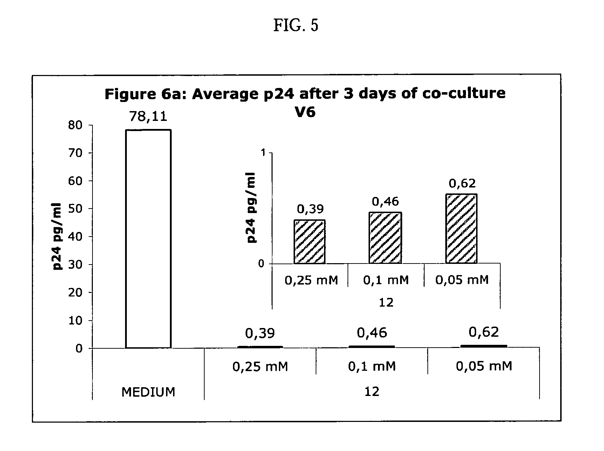

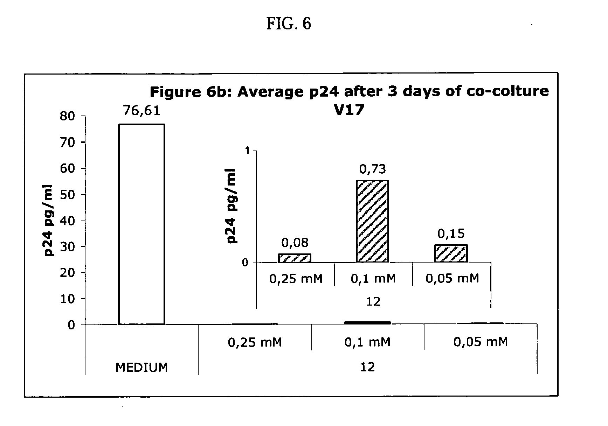

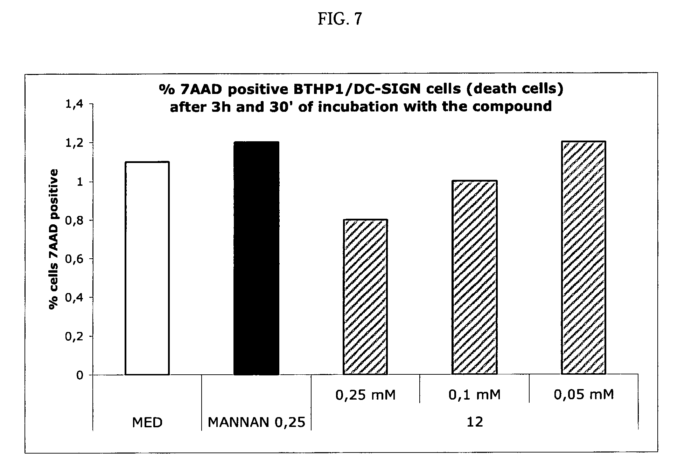

**Fig. 1** shows the results of the trans-infection of CD4+ T lymphocytes by BaL-virus after exposure to BTHP1/DC-SIGN+ cells preincubated with the virus and with variable quantities of compound **12.** The results are averages for lymphocytes donated by three random donors. **Fig. 2** shows the results of the trans-infection of CD4+ T lymphocytes assays for compound **2b** and **12.** **Fig. 3** shows the results of the trans-infection of CD4+ T lymphocytes with BaL-virus for compound **12** and **2b** when the experiment is performed washing the B-THP-1/DC-SIGN+ cells after incubation with the inhibitors and before incubation with the virus. **Fig. 4** shows the results of the trans-infection of CD4+ T lymphocytes obtained from two different donors by BaL-virus after exposure to BTHP1/DC-SIGN+ cells preincubated with the virus and with variable quantities of compound **12.** Donor P1 is genetically not susceptible to HIV infection, donor P2 is genetically susceptible to HIV infection **Fig. 5** shows the results of the trans-infection of CD4+ T lymphocytes by HIV-1 strain V6 after exposure to BTHP1/DC-SIGN+ cells preincubated with the virus and with variable quantities of compound **12.** The results are averages for lymphocytes donated by three random donors. **Fig. 6** shows the results of the trans-infection of CD4+ T lymphocytes by HIV-1 strain V17 after exposure to BTHP1/DC-SIGN+ cells preincubated with the virus and with variable quantities of compound **12.** The results are averages for lymphocytes donated by three random donors. **Fig. 7** shows the percentage of 7-AAD positive BTHP1/DC-SIGN+ cells in the absence or in the presence of the inhibitors.

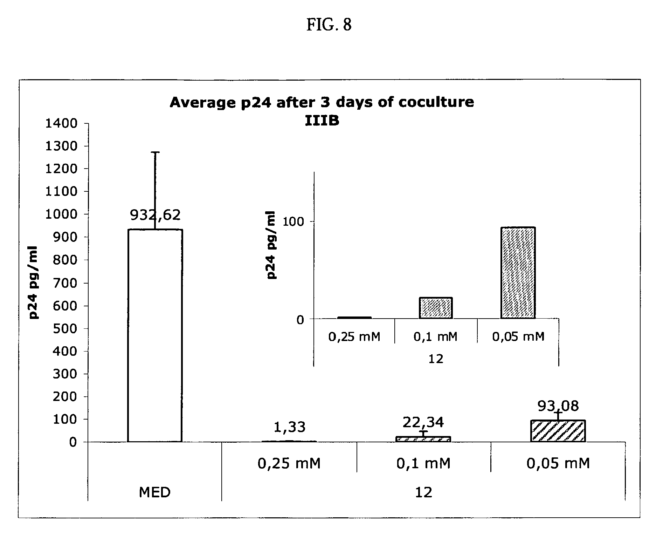

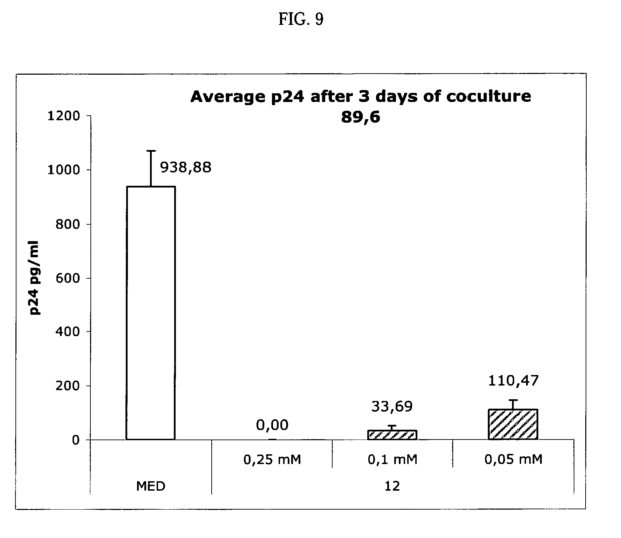

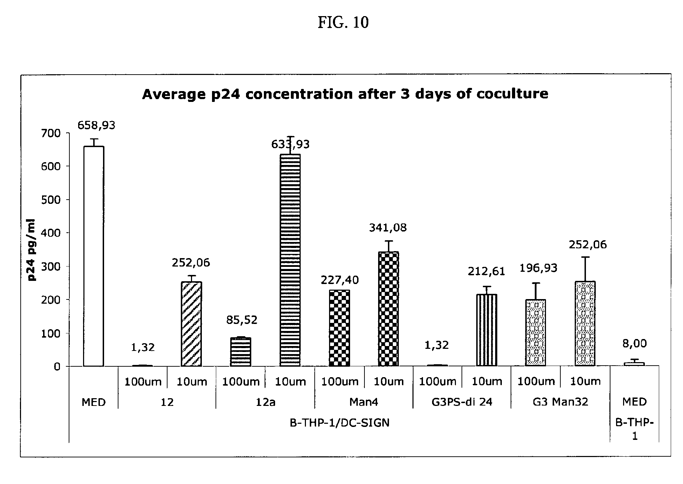

**Fig. 8** shows the results of the trans-infection of CD4+ T lymphocytes by HIV-1 strain IIIB after exposure to BTHP1/DC-SIGN+ cells preincubated with the virus and with variable quantities of compound **12.** **Fig. 9** shows the results of the trans-infection of CD4+ T lymphocytes by HIV-1 strain 89,6 (dual tropic) after exposure to BTHP1/DC-SIGN+ cells preincubated with the virus and with variable quantities of compound **12.** **Fig. 10** shows the results of the trans-infection of CD4+ T lymphocytes by HIV Bal after exposure to BTHP1/DC-SIGN+ cells preincubated with the virus and with variable quantities (10 and 100 µM) of compounds **12,** analogue tetravalent mannose **(Man4),** analogue tetravalent pseudo-dimannose **(12a),** boltorn third generation dendrimers mannosylated **(G3Man32)** and ps-diMannosylated **(G3PS-di24)** which is a third generation dendrimer based on 2,2'-bis(methoxyol)propanoic acid (bis-MPA).

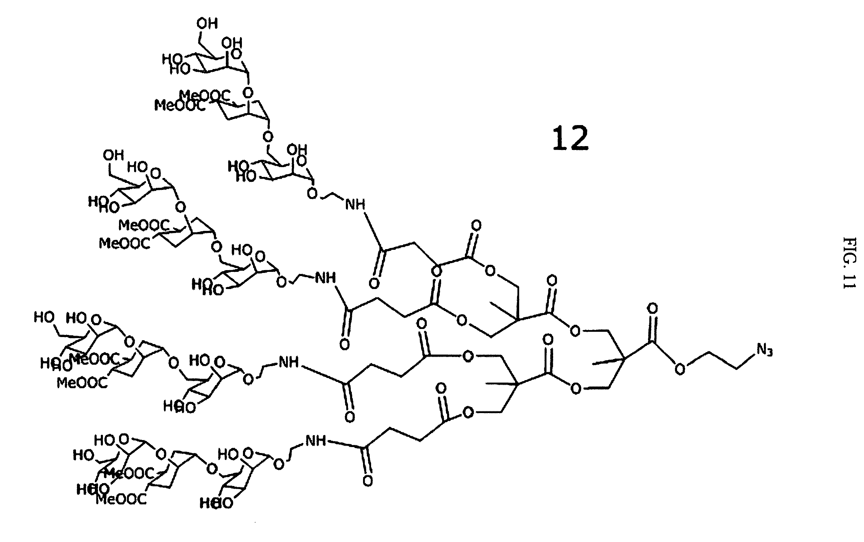

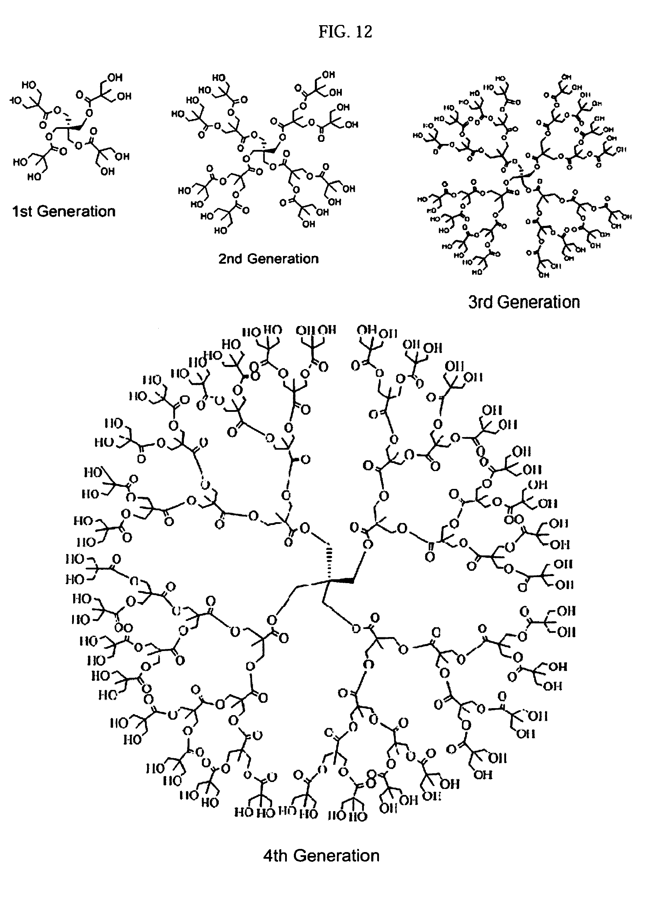

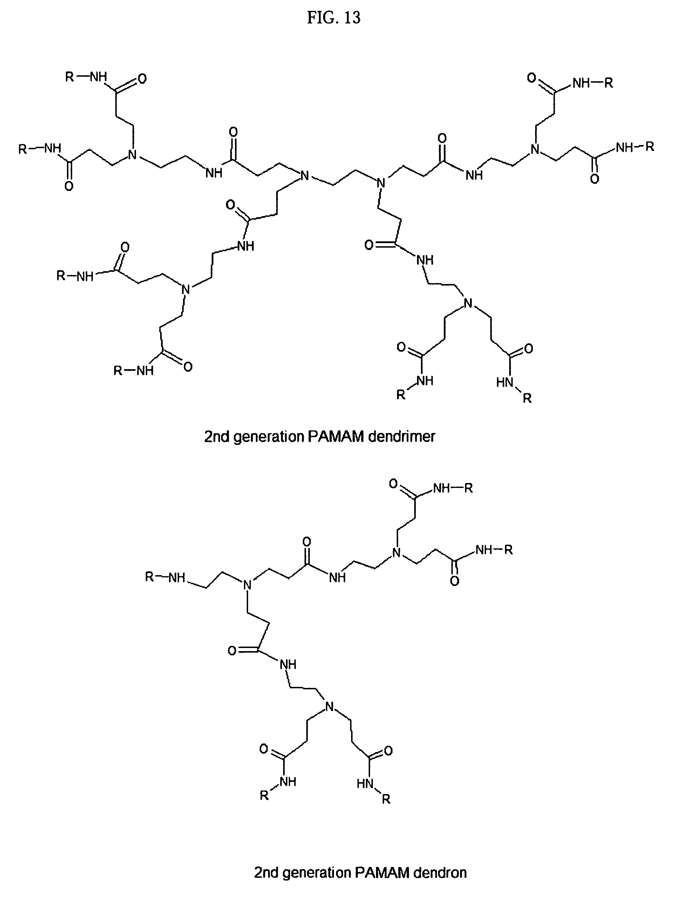

**Fig. 11** shows a preferred compound of the invention **12** (SARA 133). **Fig. 12** shows some 2,2'-bis(methoxyol)propanoic acid (bis-MPA) based dendrimers, while **Fig. 13** shows poly(amidoamine) (PAMAM) based dendrons and dendrimers within the present invention.

### DETAILED DESCRIPTION OF THE INVENTION

As for the first object of the invention, there are provided compounds of general formula (I):

**[X-Y]_{az}-Z (I)**

wherein **X** is a saccharidic or pseudosaccharidic unit, **Y** is a linker, **Z** is a scaffold and **a_{z}** is the functionality of the scaffold **Z,** i.e. the number of **X** unit it can bind, which is comprised between 1 and 150.
In particular, according to the present invention, **X** is a **M'-D-M** group, wherein:
**M'** is a saccharidic or pseudo-saccharidic non-reducing end unit, that is to say a sugar residue or a sugar residue having one or more modified ring substituents, respectively, which is not capable of reducing the oxidizing agents such as Tollens reagents (AgO), of formula 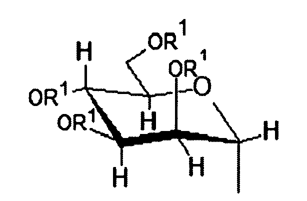 wherein R¹ is H,
   **D** is of formula 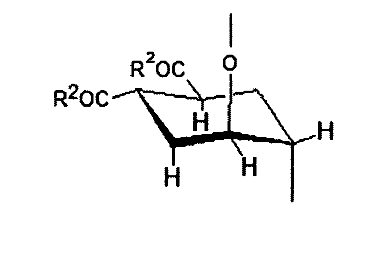 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group may be a C3-C6 saturated or partially unsaturated cyclic- or heterocyclic ring or a C5-C6 aromatic or heteroaromatic ring, which cyclic or heterocyclic, aromatic or heteroaromatic ring may be optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH), wherein preferred **A** groups are cyclopropane, cyclopentane, cyclohexane, phenyl, thiazole, imidazole, oxazole; or wherein R³ may be any of the **A** groups above disclosed; and wherein
   **M** is -(O-CH₂-CH₂)ₙ-Q or -(O-CH₂-CH₂-CH₂)ₙ-Q, wherein **Q** is an heteroatom which is bound to the **Z** scaffold through the **Y** linker and **n** is 1 to 3, so that the resulting **X** moiety may have any of the following general formulas: 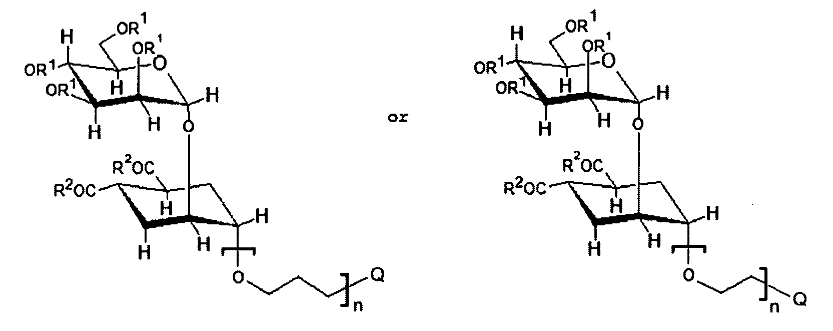 wherein R¹, R², n and **Q** have the above defined meanings; or wherein
   **M** is of formula **K**-O-(CH₂-CH₂)ₙ-**Q**, wherein **K** is a saccharidic residue selected from the group comprising a mannose, glucose, fructose, ribose, xylose or a galactose residue and **Q** and n are as above disclosed.

In a preferred embodiment, within formula (I), **K** is a mannose residue, thus **M** results any of the following: 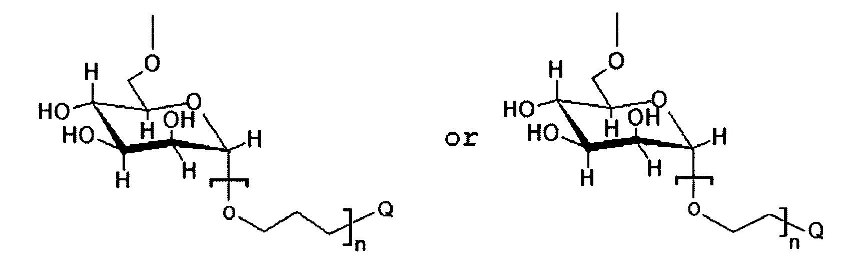 wherein **Q** and n are above disclosed and the resulting **X** moiety thus has any of the following formula: 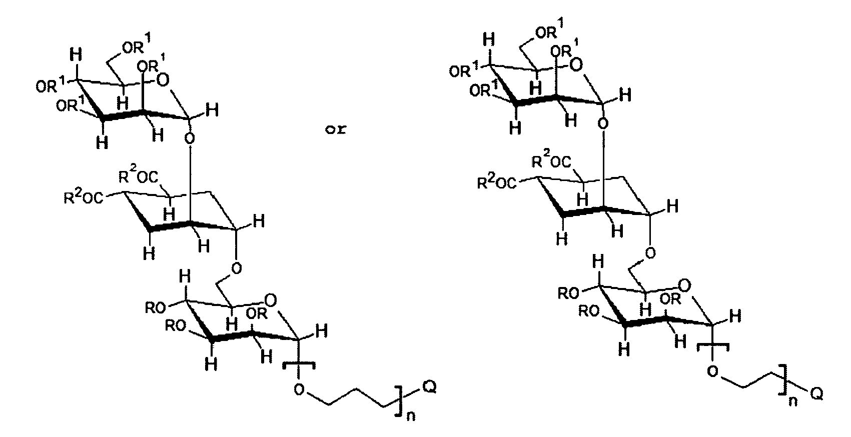 wherein R is H and R¹, R², **Q** and **n** have the same meaning as above disclosed.

Within formula (I), **Y** is a bifunctional linker, which is capable of binding the **X** moiety to one end and the **Z** scaffold to the other end; in particular, it can be a single bond, so that the scaffold **Z** is directly linked to each **X** moiety or **Y** may be an heteroatom, an amino acidic residue selected from a glycine, an alanine, a valine, a cysteine, a threonine, a serine, a proline, a methionine or a lysine residue or **Y** is a succinic acid or a diglycolic acid residue.

In the above disclosure and within the present invention, unless otherwise provided, the term heteroatom refer to any atom other than carbon or hydrogen and preferable refers to nitrogen, oxygen or sulphur; accordingly, heterocycle, heterocyclic ring or heteroaromatic ring refer to cycles, cyclic ring or aromatic ring, respectively, which comprise one or more heteroatoms. For instance, heterocycle or heterocyclic ring include tetrahydrofuran, tetrahydrothiophene, pyrrolidine, imidazolidine, pyrazolidine, while heteroaromatic rings include, for instance, furan, thiophene, pyrrole, imidazole, pyridine, pyrimidine, oxazole, isoxazole, pyrazole, etc.

According to an embodiment of the invention, preferred compounds are those of formula (I) above, wherein R¹ is H, **M** is -O-CH₂-CH₂-NH-, **a** is 1 and **Y** is a succinic acid residue.

According to a more preferred embodiment, in particular, the compounds of the invention are those wherein R² is methoxy (-OCH₃) or is -NH-R³, wherein R³ is an **A** group selected among cyclopropan, cyclohexil, phenyl, benzyl or benzyl meta or para substituted with methoxy (-OCH₃), nitro (-NO₂), carboxy (-COOH), methylester (-COOCH₃), methyl (-CH₃), methoxy (-OCH₃), amino (-NH₂), isopropyl (-CH(CH₃)₂) or R³ is 1,3, thiazole or methyl substituted 1,3-thiazole; alternatively, R³ is a methyl group (CH₃) substituted with the above mentioned **A** groups.

Thus, the preferred **X** moieties are those of the following **Table I:**
**Table I** 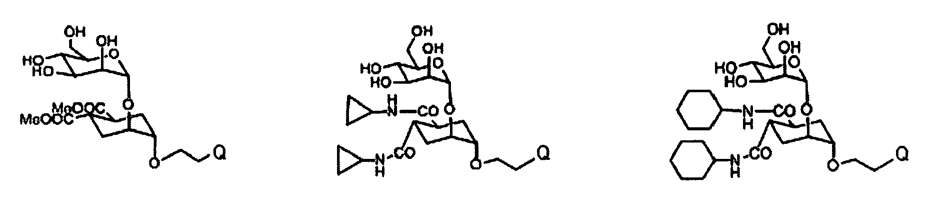 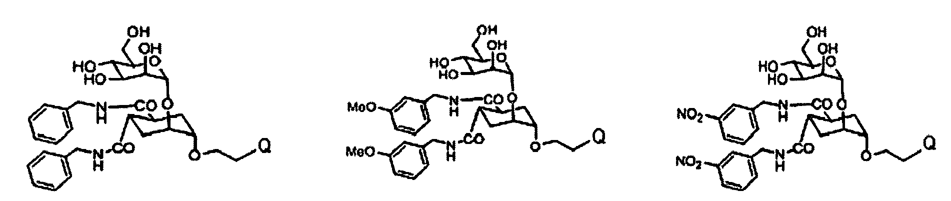 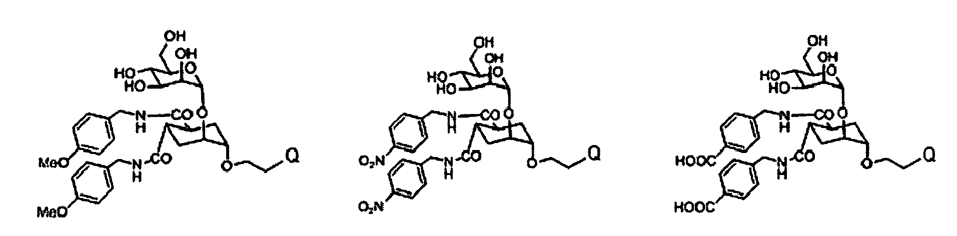 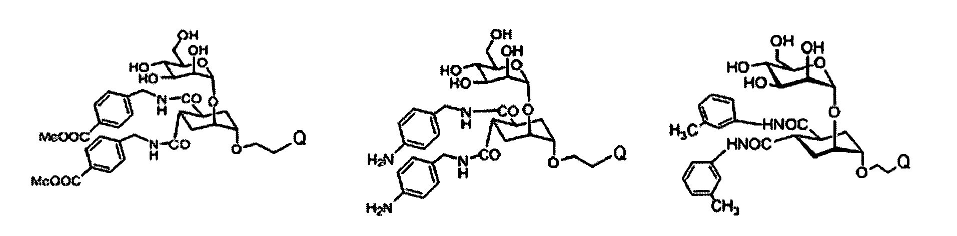 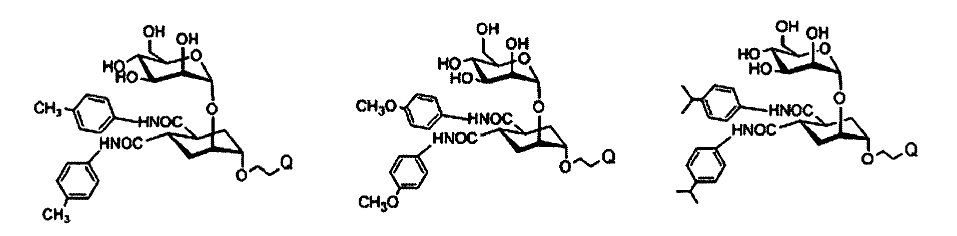 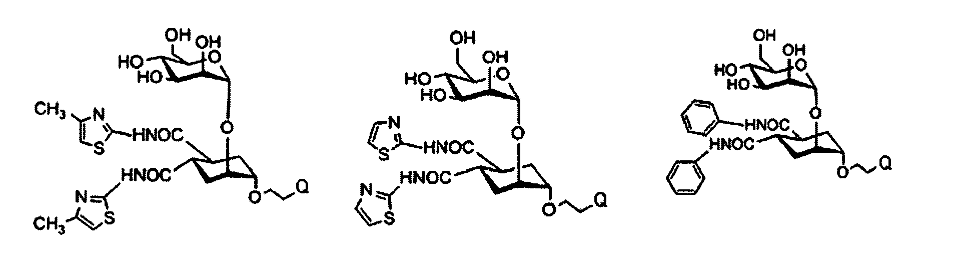 wherein **Q** is an heteroatom as above disclosed, preferably nitrogen, which is bond to the **Z** scaffold through the **Y** linker.

As for the scaffold moiety, within the compounds of formula (I), **Z** may be selected among liposomes, nanoparticles, polymers, dendrimers or dendrons. Liposome, in particular, may be composed of naturally-derived phospholipids with mixed lipid chains like phosphatidylethanolamine or of surfactant components like dioleoylphosphatidylethanolamine (DOPE), etc. In particular, they consist in phospholipid vesicles that have a bilayer membrane structure similar to that of the biological membrane and an internal aqueous phase. Their amphiphilic nature enables liposomes to transport hydrophilic drugs entrapped within their aqueous interior and hydrophobic drugs dissolved into the membrane. Moreover, the liposome surface can be modified with ligans and/or polymers to increase drug delivery specifity (see, for instance, Torchilin, V. P. Nat. Rev. Drug Discov. 2005, 4, 145-160).

Inorganic nanoparticles, in particular, may be a type of multivalent compound which have a central core composed by inorganic material (metals, ceramic, semiconductor nanocrystals, etc.) that define their physical properties. They may be made of gold, silver, Fe₂O₃, metal alloys, etc. while the core is surrounded by a functionalized linker or ligands (aliphatic chain, PEG, dextran or mixture thereof) to increase drug delivery specifity (Parak, W. J. Chem. Soc. Rev., 2008, 37, 1896 - 1908).

Carbon nanotubes belong to the family of fullerenes and are formed of coaxial graphite sheets rolled up into cylinders, in the form of single (one graphite sheet) or multi-walled concentric nanotubes. Carbon nanotubes can also be rendered water soluble by surface functionalization (Pilizu, S. et al., J. Nanosci. Nanotechnol., 2006, 6, 1883-1904).

Suitable polymers, which may be used as the **Z** scaffold comprise both artificial and natural polymers. For instance, artificial polymers include poly(ethylene glycol)(PEG), poly(amidoamine), poly(propyleneamine) (POPAM), poly(propyleneimine) (PPI), etc., while natural polymers include peptides, oligopeptides and sugars, such as, for instance, dextran, cellulose, etc.

Other suitable scaffolds may be aromatic based, ethylene diamine based or propylene diamine based.

In particular, in a preferred embodiment, the **Z** scaffold is a polyester dendron based on 2,2'-bis(methoxyol)propanoic acid (bis-MPA), such as the dendron 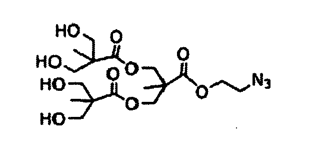 or is a first or second or higher generation dendrimer based on 2,2'-bis(methoxyol)propanoic acid (bis-MPA) such as

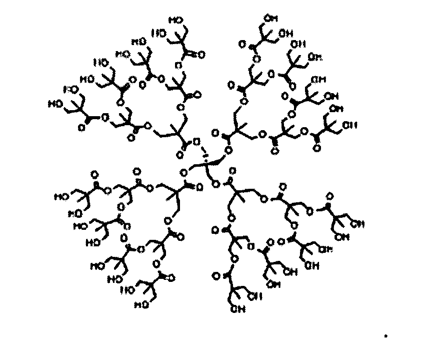

Alternatively, **Z** is a poly(amidoamine) (PAMAM) dendron or dendrimer (see, for instance, at Fig. 13)

Within the compounds of formula (I), the one wherein when the dendrimer is the third generation dendrimer based on 2,2'-bis(methoxyol)propanoic acid (bis-MPA): 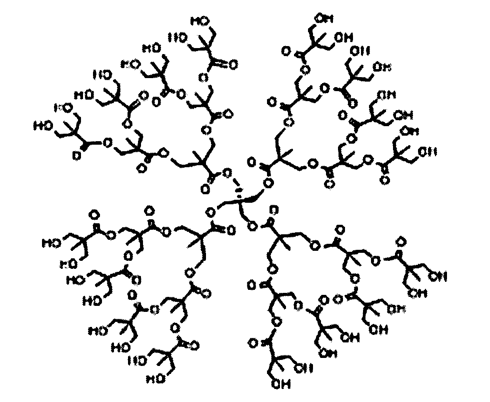 and the **X** moiety is 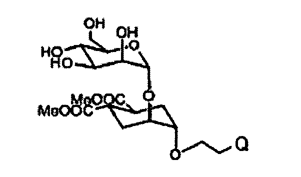 is excluded from the present invention.

According to a second aspect of the first object, the compounds of the invention have the general formula (Ia):

**[X-Y]_{aw}-W (Ia)**

wherein **X** is a saccharidic or pseudo-saccharidic unit, **Y** has the same meaning as above disclosed and wherein **aw** is the functionality of the **W** dendron scaffold and it is comprised between 1 and 100. In particular, according to the present invention, **X** is a **M'-D-M** group, wherein:
**M'** is a saccharidic or pseudo-saccharidic non-reducing end unit of formula 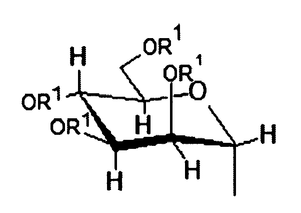 wherein R¹ is H,
   **D** is of formula 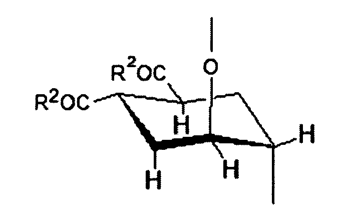 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group may be a C3-C6 saturated or partially unsaturated cyclic- or heterocyclic ring or a C5-C6 aromatic or heteroaromatic ring, which cyclic or heterocyclic, aromatic or heteroaromatic ring may be optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH), wherein preferred **A** groups are cyclopropane, cyclopentane, cyclohexane, phenyl, thiazole, imidazole, oxazole; or wherein R³ may be any of the **A** groups above disclosed; and wherein
   **M** is -(O-CH₂-CH₂)ₙ-Q or -(O-CH₂-CH₂-CH₂)ₙ-Q, wherein **Q** is an heteroatom which is bound to the **W** scaffold through the **Y** linker and **n** is 1 to 3, so that the resulting **X** moiety may have any of the following general formulas: 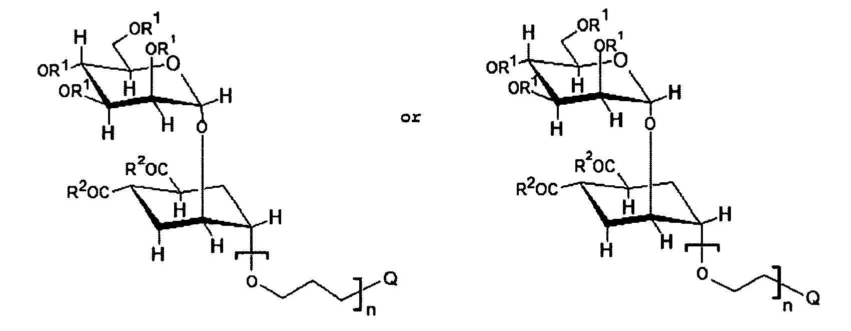 wherein R¹, R², n and **Q** have the above defined meanings or
   **M** is of formula **K**-O-(CH₂-CH₂)ₙ-**Q**, wherein **K** is a saccharidic residue selected from the group comprising a mannose, glucose, fructose, ribose, xylose or a galactose residue, **n** is 1 to 3 and **Q** is an heteroatom bond to the **W** dendron scaffold through the **Y** linker, so that the **X** resulting moiety is: 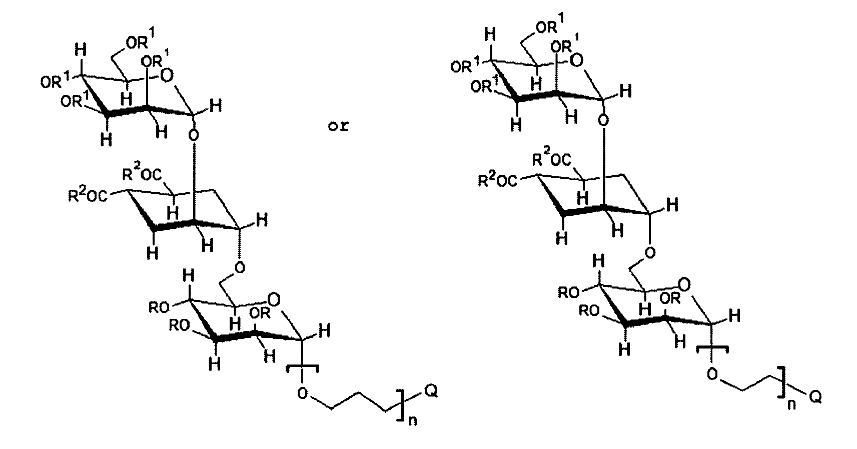 wherein R is H.

Within formula (Ia), **Y** is a bifunctional linker, which is capable of binding the **X** moiety to one end and the dendron **W** scaffold to the other end; in particular, it can be a single bond, so that the dendron scaffold **W** is directly linked to each **X** moiety or Y may be an heteroatom, an amino acidic residue selected from a glycine, an alanine, a valine, a cysteine, a threonine, a serine, a proline, a methionine or a lysine residue, a succinic acid or a diglycolic acid residue. Accordingly, preferred compounds within formula (Ia) above are compounds of formula 12 or **12a** below: 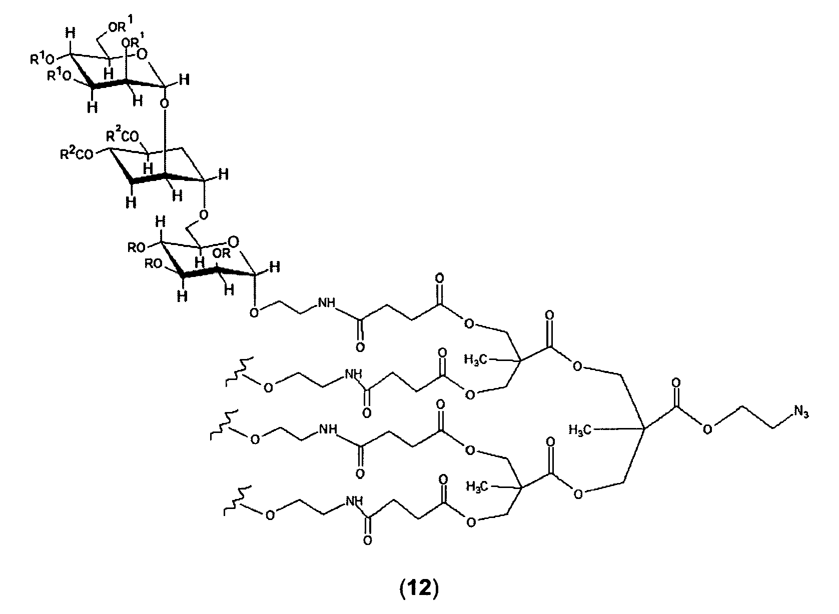 wherein R and R¹ is H and R² is -OCH₃ and 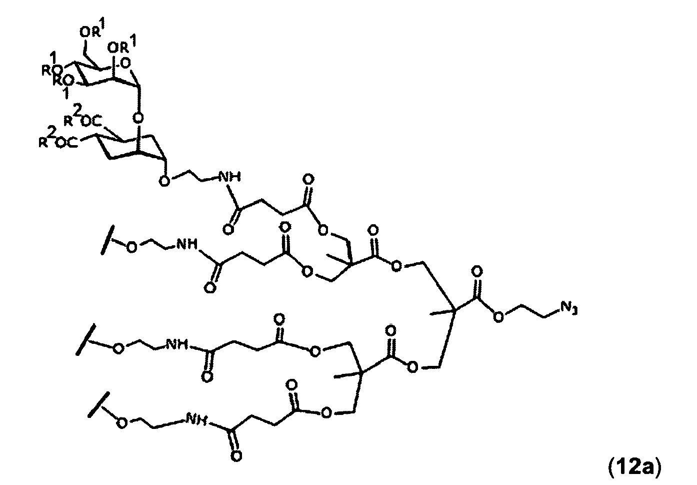 wherein R¹ is H and R² is -OCH₃.

As per a second aspect of the first object, the compounds of the invention are those of formula **(Ib):**

**[X-Y]ₐⱼ-J (Ib)**

wherein **X** is a saccharidic or pseudosaccharidic unit of formula **M-D-M',** wherein
**M'** is of formula 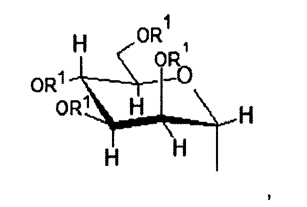 wherein R¹ is H,
**D** is of formula 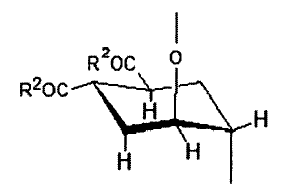 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group is a C3-C6 saturated or partially unsaturated cyclic- or heterocyclic ring or a C5-C6 aromatic or heteroaromatic ring, optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH); or R³ is an **A** group as above defined; and **M** is of formula 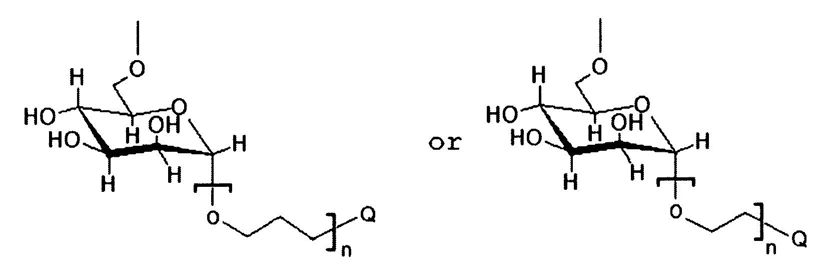 wherein **Q** is an heteroatom selected from oxygen, sulphur and nitrogen linked to a **J** scaffold through a **Y** linker and **n** is 1 to 3; or wherein
R² is -N-R³₂ as above disclosed and **M** is -(O-CH₂-CH₂)ₙ-Q or -(O-CH₂-CH₂-CH₂)ₙ-Q, wherein **Q**, **Y** and n are as above disclosed, wherein each R³ may be independently H, a C1-C8 linear or branched, saturated or unsaturated alkyl chain, optionally substituted with an above disclosed **A** group; **Y** is a linker selected from an amino acidic residue or a succinic acid or diglycolic acid residue, **J** is a first or second or higher generation dendrimer based on 2,2'-bis(methoxyol)propanoic acid (bis-MPA) or a poly(amidoamine) (PAMAM) based dendrimer and **aJ** is the functionality of the scaffold **J** and is comprised between 1 and 150.

Within formula **(Ib)** above, **M** is preferably 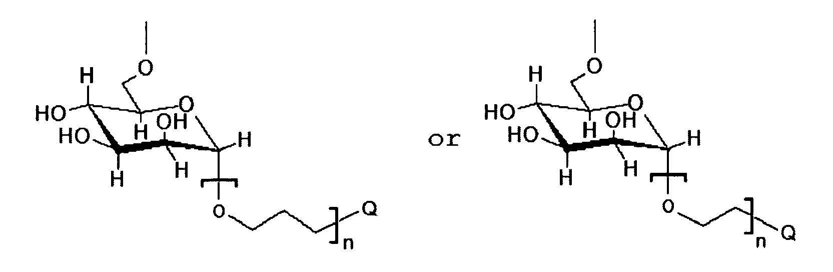 and the resulting **X** moiety has the following formula 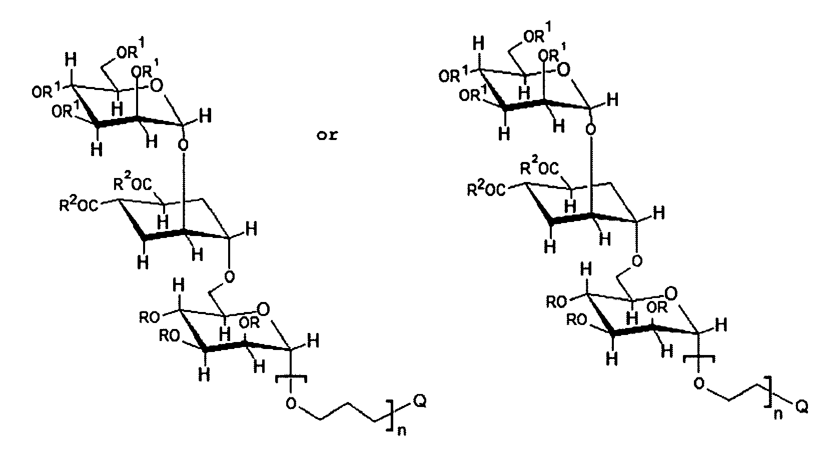 wherein R, R¹, R², **Q** and n having the same meaning as above disclosed.

In particular, in a preferred embodiment, said dendrimer based on 2,2'-bis(methoxyol)propanoic (bis-MPA) is a first or second generation or higher generation bis(methoxyol)propanoic (bis-MPA) based dendrimers (see, for instance, at Fig. 12).

According to the above, a preferred compound within formula **(Ib)** is below compound **15** 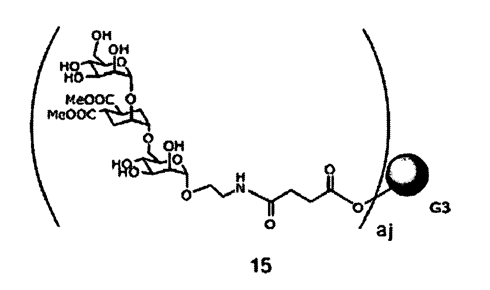 prepared as disclosed in Example 3, wherein R¹ is H, R² is methoxy (-OCH₃), R is H, n is 1, **Q** is nitrogen, **Y** is a succinic acid residue and G3 is the **J** scaffold, which is a third generation 2,2'-bis(methoxyol)propanoic (bis-MPA) based dendrimer.

Alternatively, the dendrimer scaffold **J** may be a poly(amidoamine) (M) based dendrimer (see, for instance, at Fig. 13).

According to a second object of the invention, there is provided a process for the preparation of the compounds of the invention.

In particular, said method includes the step of providing an hydroxyl-protected **M** moiety, which first shall be deprotected at the 6-OH group. Then, a suitable precursor of the **D** moiety, such as, for instance, an epoxyde, is reacted in order to give the **M-D** dimer. The dimer is then added to a solution of the scaffold, which already bears the linker **Y** moiety, in order to obtain a compound of the invention.

In order to prepare the **M'-D-M** trimeric **X** moiety, the **M-D** dimer above obtained is further coupled to a suitably protected **M'** moiety thus yielding the trimer, which is then treated to remove the protecting group of both the **M** and **M'** moieties and further deprotected and optionally reduced to provide the final **M'-D-M** trimer.

Finally, to a solution of the scaffold already bearing the linker **Y** moiety, there is added a suitable amount of the **M'-D-M** trimer.

In a third object of the invention, there is disclosed the use of the compounds of formula (I) as antiinfective and anti-viral medicaments against HIV virus (Human Immunodeficiency Virus), Dengue virus, Ebola virus, Hepatitis C virus, SARS and *Mycobacterium tuberculosis.*

In another aspect of said third object, there is provided the use of the compounds of formula **(Ia)** as antiinfective and anti-viral medicaments against HIV virus (Human Immunodeficiency Virus), Dengue virus, Ebola virus, Hepatitis C virus, SARS and *Mycobacterium tuberculosis* infections.

It is a further aspect of the present invention, the use of the compounds of formula **(Ib)** as antiinfective and anti-viral medicaments against HIV virus (Human Immunodeficiency Virus), Dengue virus, Ebola virus, Hepatitis C virus, SARS and *Mycobacterium tuberculosis* infections.

In a preferred embodiment, there is disclosed the use of the compounds of formula (I) having the **X** moieties of Table I or the one of formula 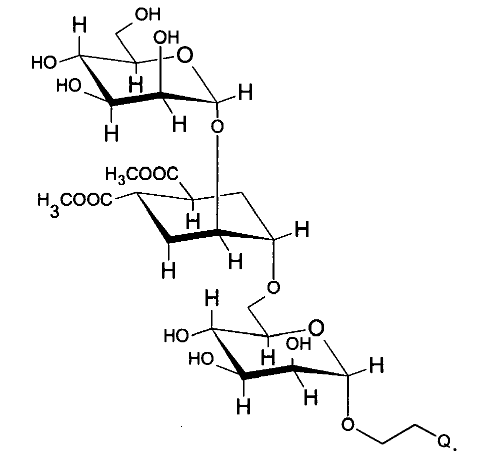 as antiinfective and anti-viral medicaments against HIV virus (Human Immunodeficiency Virus), Dengue virus, Ebola virus, Hepatitis C virus, SARS and *Mycobacterium tuberculosis* infections.

In a still preferred embodiment of the invention, there is provided the use of the compounds **12** and **12a** for the treatment and/or prevention of HIV (Human Immunodeficiency Virus), Dengue, Ebola, Hepatitis C, SARS and *Mycobacterium tuberculosis* infections.

In a still further embodiment, the invention provides the use of the compound of formula 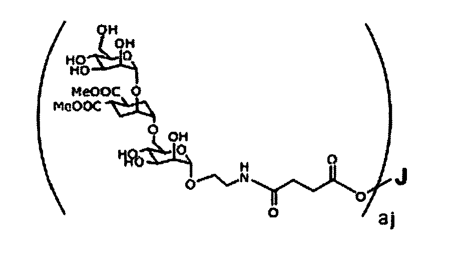 wherein **aj** is 32 and **J** is a third generation 2,2'-bis(methoxyol)propanoic acid (bis-MPA) based dendrimer for the treatment and/or prevention of HIV (Human Immunodeficiency Virus), Dengue, Ebola, Hepatitis C, SARS and *Mycobacterium tuberculosis* infections.

For the purposes of a forth embodiment of the present invention, the disclosed compounds may be formulated in a suitable effective amount together with suitable pharmaceutically acceptable excipients according to the specific form of administration and/or additives selected in the group comprising diluent, solvents, bulking agents, fillers, reological modifier, stabilizers, binders, lubricants, disintegrant, preservatives, pH adjusting agents, buffers, antioxidant, chelating agents, plasticizer, polymers, emulsifiers, edulcorants, flavoring agents, alone or in combination thereof, to give a pharmaceutical preparation as per a further embodiment of the invention.

The preparations of the present invention may be administered by various route of administration, such as, for instance, oral, including buccal or sublingual administration; rectal; nasal; transdermal; vaginal; or parenteral, including subcutaneous, intramuscular, intravenous or intradermal route or topically.

In particular, for oral administration, they can be prepared in the form of hard or soft capsules, tables, powders or granules; solutions syrups or suspensions in aqueous or non-aqueous liquids; edible foams or whips; or either oil-in-water or water-in-oil liquid emulsions. Alternatively, for nasal administration sprays or drops may be prepared; for vaginal administration they may be prepared as pessaries, tampons, creams, gels, pastes, foams or spray formulations; for parenteral administration, aqueous and non-aqueous sterile injectable solutions or suspensions may be prepared.

In a particularly preferred embodiment, the compounds of the invention are administered topically, for instance, in the form of ointments, creams, suspensions, lotions, powders, solutions, pastes, gels, sprays, aerosols or oils. The present invention will be further better detailed in the following Experimental Section.

### EXPERIMENTAL SECTION

### Synthesis- General Information

NMR spectra were recorded at 25°C and 30 °C with Bruke r spectrometers. Chemical shifts ¹H and ¹³C NMR spectra are expressed in ppm relative to TMS or to DSS for spectra recorded in D₂O. The cyclohexanediol moiety is numbered as in Fig. 5. Subscripts D refer to the cyclohexanediol (ps-Man) residue of 7 and 9 and 2. Mass spectrometry was performed with a ThermoFisher LCQ apparatus (ESI ionization), or ion-trap ESI Esquire 6000 from Bruker, or an Microflex apparatus (MALDI ionization) from Bruker, or Apex II ICR FTMS (ESI ionization - HR-MS). Optical rotations [α]_{D} were measured in a 1-dm path-length cell with 1 mL capacity on a Perkin-Elmer 241 polarimeter. Thin layer chromatography (TLC) was carried out with precoated Merck F254 silica gel plates. Unless otherwise specified the TLC were revealed with molibdic reagent. Flash chromatography (FC) was carried out with Macherey-Nagel silica gel 60 (230-400 mesh). Final dendritic compounds were purified by size exclusion chromatography using sephadex LH20 from GE Healthcare Life Science. Solvents were dried by standard procedures and reactions requiring anhydrous conditions were run under nitrogen. Known compounds have been previously described **8, 6** (Mari, S.; Posteri, H.; Marcou, G.; Potenza, D.; Micheli, F.; Cañada, F. J.; Jiménez-Barbero, J.; Bernardi, A. Eur. J. Org. Chem. 2004, 5119-5225) **4** (Lingrong Gu et al, Biomacromolecules, 2008, 9, 2408-2418) **5** (Lingrong Gu et al, Biomacromolecules, 2008, 9, 2408-2418; Pathak, A. K. et al, Carbohydrate Research, 2004, 339, 683-691) and **13** (Arce, E. et al. Bioconjugate Chem., 2003, 14, 817-823).

### EXAMPLE 1

### Synthesis of compound 12 (SARA 133)

### 1) Synthesis of the (2-azidoethyl)-2,3,4-tri-O-acetyl-α-D-mannopyranoside

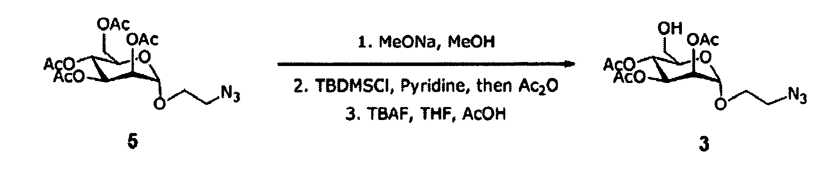

To a solution of the azido-ethyl derivative 5 (3.26 g, 7.81 mmol, 1 eq) in dry MeOH (53 mL), freshly prepared MeONa 1 M in dry MeOH (1.25 mL) was added. The reaction was stirred at room temperature, monitoring by TLC (1:1 Hex:EtOAc). After 20 min the reaction mixture was neutralized with an acidic resin (Amberlite IRA 120 H+) and the solvent was evaporated at reduced pressure, obtaining 1.95 g (quantitative) of the azidoethylmannoside, as a white solid, which was used without any other purification. ¹H-NMR (D₂O, 400 MHz) δ (ppm): 4.95 (d, 1 H, H₁, J₁₋₂= 1.6 Hz), 4.01 (dd, 1H, H₂, J₂₋₃= 3.2 Hz), 3.98-3.92 (m, 1H, H_{7B}), 3.95-3-91 (m, 1 H, H_{6B}), 3.87 (dd, 1 H, H₃, J₃₋₄= 9.4 Hz), 3.82-3-77 (m, 1H, H_{6A}), 3.78-3.73 (m, 1 H, H_{7A}), 3.72-3.68 (m, 2H, H₄, H₅), 3.58 (ddd, 1H, H_{8B}, J_{gem}= 13.6 Hz, J_{8B-7} = 6.4 Hz, J_{8B-7'} = 3.2 Hz), 3.52 (ddd, 1 H, H_{8A}, J_{8A-7} = 6.0 Hz, J_{8A-7'} = 3.2 Hz). ¹³C-NMR (D₂O, 100 MHz) δ (ppm): 99.6 (C1), 72.7 (C4), 70.2 (C3), 69.7 (C2), 66.5 (C5), 66.1 (C7), 60.7 (C6), 50.0 (C8). MS (ESI) [C₈H₁₅N₃O₆Na]⁺= 272.6, [C₈H₁₅N₃O₆K]⁺= 288.4. ${\left[α\right]}_{D}^{20} = + 55.64 \left(c: 1.2,MeOH\right) .$ To a solution of the azidoethylmannoside (1.95 g, 7.81 mmol, 1 eq) in dry pyridine (18 mL) at 0 °C a solution of TBDMSCI (1.41 g, 9.37 mmol, 1.2 eq) in dry pyridine (8 mL) was added. The reaction was stirred at 0°C for one hour, then warmed at room tem perature. The reaction, monitored by TLC (8:2 CHCl₃:MeOH), was complete after 4 hours. Then Ac₂O (4.43 mL, 46.9 mmol, 6 eq) was added and the reaction mixture was stirred overnight. The solvent was then evaporated at reduced pressure; the crude was diluted with EtOAc, washed with 3N HCl, then with saturated NaHCO₃ solution and with water till neutral pH. The organic phase was dried over anhydrous sodium sulfate and the solvent was evaporated at reduced pressure. The crude was purified by flash chromatography (8:2 Hex:EtOAc) obtaining 3.10 g of a colourless sticky oil (yield 81%) ${\left[α\right]}_{D}^{20} = + 43.09$ (c: 0.955, CHCl₃). ¹H-NMR (400 MHz, CDCl3) δ (ppm): 5.36 (dd, 1 H, H₃, J₃₋₂= 3.6 Hz, J₃₋₄= 10.0 Hz), 5.28 (t, 1H, H₄, J₄₋₅= 10.0 Hz), 5.24 (dd, 1 H, H₂, J₂₋₁=2.0 Hz), 4.84 (d, 1H, H₁), 3.92-3.81 (m, 2H, H_{7B}, H₅), 3.70 (t, 2H, H₆, J₆₋₅= 4 Hz), 3.68-3.60 (m, 1H, H_{7A}), 3.51-3.38 (m, 2H, H₈), 2.13 (s, 3H, CH₃CO), 2.03 (s, 3H, CH₃CO), 1.99 (s, 3H, CH₃CO), 0.90 (s, 9H, C(CH₃)₃), 0.06 (s, 3H, CH₃Si), 0.05 (s, 3H, CH₃Si).¹³C-NMR (100 MHz, CDCl₃) δ (ppm): 170.2 (COCH₃), 170.1 (COCH₃), 169.8 (COCH₃), 97.6 (C1), 71.9 (C5), 69.8 (C2), 69.4 (C3), 66.9 (C7), 66.6 (C4), 62.4 (C6), 50.5 (C8), 25.9 (C(CH₃)₃), 21.0 (CH₃CO), 20.9 (CH₃CO), 20.8 (CH₃CO), 18.4 (C(CH₃)₃), -5.2 (SiCH₃), -5.3 (SiCH₃). HRMS (ESI) : Calculated for [C₂₀H₃₅N₃O₉SiNa]⁺ = 512.20348 Experimental = 512.20261.

To a solution of the silylmannopyranoside previously obtained (3.10 g, 6.33 mmol, 1 eq) in dry THF (63 mL) at 0°C under nitroge n atmosphere 1.81 mL of acetic acid were added slowly (31.65 mmol, 5 eq). The solution was then warmed at room temperature and 7.60 mL (7.60 mmol, 1.2 eq) of TBAF 1 M in dry THF were added.

The reaction was stirred overnight at room temperature. After completion (TLC 2:1 ETP:EtOAc) the solution was diluted with brine and then extracted with EtOAc. The organic phases were collected, dried over Na₂SO₄, and the solvent was evaporated at reduced pressure. The crude was purified by flash chromatography (4:6 Hex:EtOAc) obtaining 1.71 g of pure product 3 (72%). ${\left[α\right]}_{D}^{20} = + 42.32$ (c: 1.17, CHCl₃). ¹H-NMR (400 MHz, CDCl₃) δ (ppm): 5.42 (dd, 1 H, H₃, J₃₋₂= 3.6 Hz J₃₋₄= 10.0 Hz), 5.29 (dd, 1 H, H₂, J₂₋₁=1.6 Hz), 5.25 (t, 1H, H₄, J₄₋₅= 10.4 Hz), 4.88 (d, 1 H, H₁), 3.87 (ddd, 1 H, H_{7B}, J_{gem}= 10.8 Hz J_{7B-8B}= 7.2 Hz, J_{7B-8A}= 3.6 Hz), 3.85-3.79 (m, 1 H, H₅), 3.72 (d, 1H, H_{6B}, J_{gem}= 12.8 Hz), 3.69-3.60 (m, 2H, H_{6A}, H_{7A}), 3.49 (ddd, 1 H, H_{8B}, J_{gem}= 13.6 Hz, J_{8B-7A}= 3.6 Hz), 3.42 (ddd, 1H, H_{8A}, J_{8A-7A}= 6.0 Hz), 2.14 (s, 3H, CH₃CO), 2.08 (s, 3H, CH₃CO), 2.00 (s, 3H, CH₃CO). ¹³C-NMR (100 MHz, CDCl₃) δ (ppm): 171.0 (COCH₃), 170.2 (COCH₃), 169.9 (COCH₃), 98.0 (C1), 71.1 (C5), 69.6 (C2), 68.8 (C3), 67.1 (C7), 66.5 (C4), 61.4 (C6), 50.5 (C8), 21.0 (CH₃CO), 20.9 (CH₃CO), 20.8 (CH₃CO). HRMS (ESI): Calculated for [C₁₄H₂₁N₃O₉Na]⁺ = 398.11700 Experimental = 398.11622.

### 2) synthesis of the pseudo-α(1,6)-dimannoside 7 acetylated

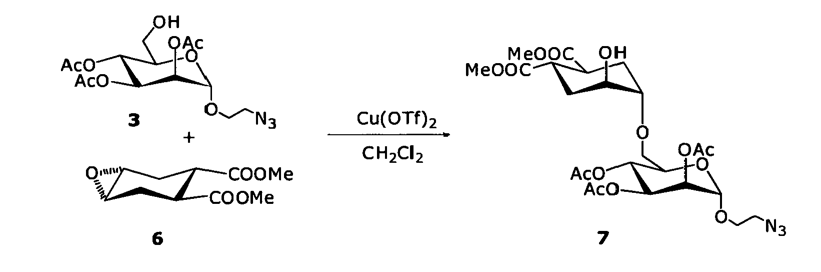

To a solution of the epoxide **6** (100 mg, 0.467 mmol, 1eq.) and the mannose derivative **3** (525 mg, 1.400 mmol, 3 eq.) in CH₂Cl₂ (1 ml) the catalyst Cu(OTf)₂ was added (17 mg, 0.047 mmol, 0.1 eq.).

The solution was stirred at room temperature under nitrogen atmosphere overnight. The reaction was followed by TLC (92:8 CH₂Cl₂: acetone). After completion the solution was diluted with a [1:1] mixture of NH₄Clₛₐₜ and NH₄OH and then extracted with EtOAc. The organic phase was washed with NH₄Clₛₐₜ till the disappearance of the light blue colour of the aqueous phase, then dried over Na₂SO₄. The solvent was evaporated at reduced pressure, giving a crude that was purified by flash chromatography (95:5 CHCl₃:acetone). 147mg of pure **7** were obtained (yield 53%). ${\left[α\right]}_{D}^{20} = + 40.0$ (c: 1.00, CHCl₃). ¹H-NMR (400 MHz, CDCl₃) δ (ppm): 5.38-5.29 (m, 2H, H_{3M}, H_{4M}), 5.26 (dd, 1H, H_{2M}, J₂₋₃ = 2 Hz, J₂₋₁ = 1.6 Hz), 4.85 (d, 1H, H_{1M}), 3.99-3.92 (m, 1H, H_{5M}), 3.89 (ddd, 1H, H_{7B}, J_{gem} =10.8 Hz, J_{7A-8} = 6.8 Hz, J_{7A-8'} = 3.6 Hz ), 3.85-3.79 (m, 1 H, D₂), 3.73 (dd, 1 H, H_{6B}, J_{gem}= 11.2 Hz, J_{6B-5}= 5.2 Hz), 3.69 (s, 3H, OCH₃), 3.69 (s, 3H, OCH₃), 3.65 (m, 1 H, H_{7A}), 3.55 (dd, 1 H, H_{6A}, J_{6A-5}= 2.4 Hz), 3.50-3.40 (m, 3H, H₈, D₁), 3.12 (dt, 1H, D₄, J_{D4-D3ax}= J_{D4-D5}= 7.6 Hz, J_{D4-D3eq}= 4.4 Hz), 3.07 (dt, 1H, D₅, J_{D5-D6ax}= 7.6 Hz, J_{D5-D6eq}= 4.4 Hz), 2.17-2.08 (m, 1 H, D₃ₐₓ), 2.10-2.02 (m, 1 H, D₆ₐₓ), 2.16 (s, 3H, CH₃CO), 2.06 (s, 3H, CH₃CO), 1.99 (s, 3H, CH₃CO), 1.91-1.83 (m, 1 H, D_{6eq}), 1.83-1.75 (m, 1 H, D_{3eq}). ¹³C-NMR (100 MHz, CDCl₃) δ (ppm): 174.57, 174.52 (COOCH₃), 170.10, 169.92 (CH₃CO), 97.62 (C1_{M}), 78.55 (C1_{D}), 70.22 (C5_{M}), 69.47 (C2_{M}), 69.07 (C3_{M}/C4_{M}). 68.13 (C2_{D}), 67.78 (C6_{M}), 67.03 (C7), 66.42 (C3_{M}/C4_{M}), 52.04 (OCH₃), 50.43 (C8), 39.61 (C5_{D}), 39.28 (C4_{D}), 30.48 (C3_{D}), 27.03 (C6_{D}), 20.88, 20.83, 20.71 (CH₃CO). HRMS (ESI): Calculated for [C₂₄H₃₅N₃O₁₄Na]⁺ = 612.20112 Experimental = 612.19995.

### 3) Synthesis of the α(1,2)α(1,6)pseudomannotriose 9

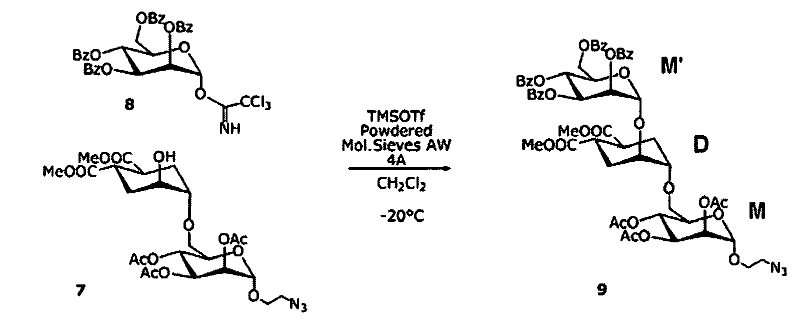

A mixture of the acceptor **7** (53.2 mg, 0.0902 mmol, 1 eq) and the donor **8** (80.2 mg, 0.1083 mmol, 1.2 eq) was co-evaporated with toluene three times, then powdered molecular sieves 4A acid washed were added; the mixture was kept under vacuum for few hours and then dissolved with dry CH₂Cl₂ (1.8 mL). After cooling at -20°C, TMSOTf (3 µl, 0.018 mmol, 0. 2 eq.) was added and the reaction mixture was stirred at that temperature. The reaction was monitored by TLC (7:3 CH₂Cl₂:EtOAc and 7:3 Toluene:EtOAc): after 20 minutes the reaction was finished.

In order to quench the reaction NEt₃ was added. The mixture was brought to room temperature and filtered over a celite pad, then the solvent was evaporated at reduced pressure obtaining 140 mg of crude as a white solid. The purification was performed by flash chromatography (8:2 Toluene:EtOAc) leading to **9** (86 mg, 82%) as a white solid. ${\left[α\right]}_{D}^{20} = - 2.16$ (c: 1.016, CHCl₃). ¹H-NMR (400 MHz, CDCl₃) δ (ppm): 8.08 (dd, 4H, H-Ar OBz J = 6 Hz), 7.99 (d, 2H, H-Ar OBz J = 8 Hz), 7.84 (d, 2H, H-Ar OBz J = 8 Hz), 7.65-7.48 (m, 3H, H-Ar OBz), 7.47-7.34 (m, 7H, H-Ar OBz), 7.32-7.22 (m, 2H, H-Ar OBz), 6.14 (t, 1H, H_{4M'}, J₄₋₃ = J₄₋₂ = 10.2 Hz), 5.88 (dd, 1 H, H_{3M'}, J₃₋₂ = 3.0 Hz), 5.70 (bs, 1 H, H_{2M'}), 5.38 (dd, 1 H, H_{3M}, J₃₋₂ = 4.0 Hz, J₃₋₄ = 10.0 Hz ), 5.34-5.23 (m, 2H, H_{2M}, H_{4M}), 5.30 (s, 1H, H_{1M'}), 4.86 (s, 1H, H_{1M}), 4.70 (dd, 1H, H_{6B M'}, J_{6B-5} = 2.4 Hz, J_{gem} = 12.0 Hz), 4.49 (dd, 1 H, H_{6A M'}, J_{6A-5} = 4.4 Hz), 4.45-4.39 (m, 1 H, H_{5M'}), 4.10 (m, 1 H, D₂), 3.99-3.92 (m, 1H, H_{5M}), 3.92-3.85 (m, 1 H, H_{7B}), 3.76 (bs, 1 H, D₁), 3.72-3.62 (m, 2H, H_{7A}, H_{6B M}), 3.73 (s, 3H, OCH₃), 3.70 (s, 3H, OCH₃), 3.56 (dd, 1 H, H_{6A M}, J_{gem} = 10.8 Hz, J_{6A-5} = 2 Hz), 3.51-3.37 (m, 2H, H₈), 3.09-2.98 (m, 2H, D₄, D₅), 2.27-1.87 (m, 4H, D₃, D₆), 2.16 (s, 3H, OCOCH₃), 2.06 (s, 3H, OCOCH₃), 2.00 (s, 3H, OCOCH₃). ¹³C-NMR (100 MHz, CDCl₃) δ (ppm): 174.85, 174.76 (COOCH₃), 170.02, 169.84, 169.75 (COCH₃), 166.06, 165.49, 165.43, 165.37 (COPh), 133.56, 133.50, 133.23, 133.15 (CH Ar), 129.84, 129.72, 128.62, 128.47, 128.32 (CH Ar), 129.22, 128.99, 128.88 (CqAr), 97.63 (C_{1M}), 96.54 (C_{1M'}), 75.14 (C_{D2}), 73.38 (C_{D1}), 70.80 (C_{2M'}), 70.15 (C_{5M}), 69.81, (C_{3M'}), 69.60 (C_{5M'}), 69.44 (C_{4M}), 69.13 (C_{3M}), 68.18 (C_{6M}), 66.99 (C₇), 66.87 (C_{4M'}), 66.41 (C_{3M'}), 62.81 (C_{6M'}), 51.97, 51.91 (COOCH₃), 50.37 (C₈), 38.95, 38.74 (C_{D4}, C_{D5}), 27.44, 27.28 (C_{D3}, C_{D6}), 20.83, 20.77, 20.67 (OCOCH₃). MS (ESI) : [C₅₈H₆₁N₃O₂₃Na]⁺ = 1190.6

### 4) Synthesis of the α(1,2)α(1,6)pseudomannotriose 2b

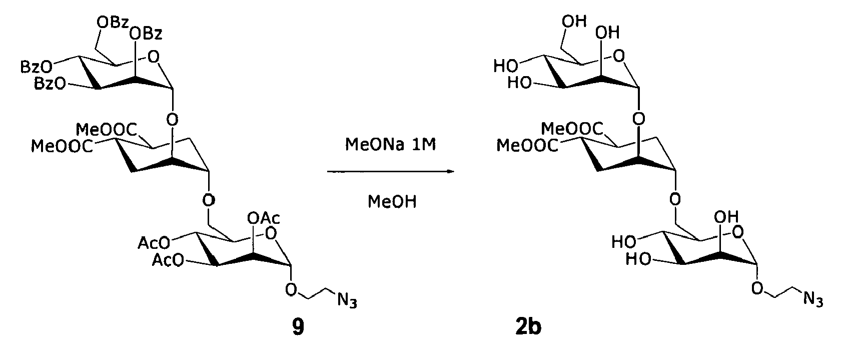

To a solution of the compound **9** (124.5 mg, 0.1066 mmol, 1 eq) in dry MeOH (2.13 mL) under nitrogen atmosphere 213 µL of MeONa 1 M were added. The reaction was stirred at room temperature monitoring by TLC (8:2 CH₂Cl₂:EtOAc, 75:25:3 CHCl₃:MeOH:H₂O). After 20 minutes the reaction was finished and the solution was neutralized with Amberlite IRA 120H⁺, the resin removed by filtration and the solvent was evaporated at reduced pressure, obtaining pure 2b as a colourless oil (70.1 mg, quantitative). ${\left[α\right]}_{D}^{20} = + 59.38$ (c: 1.025, CH₃OH). ¹H-NMR (400 MHz, CD₃OD) δ (ppm): 4.94 (d, 1H, H_{1M}, J₁₋₂ = 1 Hz), 4.82 (d, 1H, H_{1M'}, J_{1'-2'} = 1.2 Hz), 4.07 (bs, 1H, D₂), 3.96-3-92 (m, 1H, H_{7B}), 3.88-3.81 (m, 4H, H_{2M}, H_{2M'}, H_{6B M}, H_{6B M'}), 3.78-3.74 (m, 2H, D₁, H_{6A M'}), 3.74-3.67 (m, 4H, H_{6A M}, H_{7A}, H_{3M}, H_{3M'}), 3.65 (s, 3H, OCH₃), 3.65 (s, 3H, OCH₃), 3.66-3.61 (m, 4H, H_{4M'}, H_{5M'}, H_{5M}, H_{4M}), 3.43 (t, 2H, H₈, J₇₋₈ = 4.8 Hz), 3.00 (dt, 1H, D₅, J_{D5-D4}=J_{D5-D6ax} =12.8 Hz, J_{D5-D6eq}= 3.6 Hz), 2.84 (dt, 1 H, D₄, J_{D4-D3ax} =11.6 Hz, J_{D4-D3eq}= 3.6 Hz), 2.16 (app d, 1 H, D₆ₐₓ, J_{gem}= 13.6 Hz), 2.04 (app d, 1 H, D₃ₐₓ, J_{gem}= 14.0 Hz), 1.84 (t, 1H, D_{3eq}), 1.74 (t, 1H, D_{6eq}). ¹³C-NMR (100 MHz, CD₃OD) δ (ppm): 177.25, 176.99 (COOCH₃), 101.87 (C_{1M'}), 100.35 (C_{1M}), 76.06 (C1_{D}), 75.49 (C4_{M}), 74.24 (C4_{M'}), 72.59, 72.58, 72.53 (C2_{M}, C2_{M'}, C3_{M}, C3_{M'}), 71.94 (C2_{D}), 70.00 (C6_{M'}), 68.59 (C5_{M}, C5_{M'}), 63.03 (C6_{M}), 52.33 (OCH₃), 51.76 (C₈), 40.39 (C4_{D}), 40.13 (C5_{D}), 28.92 (C6_{D}), 28.30 (C3_{D}). HRMS (ESI) : Calculated for [C₂₄H₃₉N₃O₁₆Na₁]⁺ = 648.22225, experimental = 648.22127 (error 1.5 ppm).

### 5) Synthesis of the α(1,2)α(1,6)pseudomannotriose 10

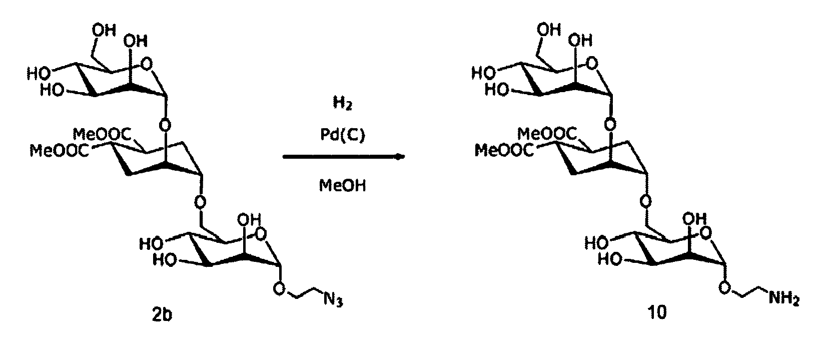

To a solution of the compound **2b** (27.4mg, 0.044 mmol, 1eq) in MeOH (2.2mL) a catalytic amount of Pd on carbon was added. The suspension was put under hydrogen atmosphere and stirred at room temperature monitoring by TLC (CHCl₃:MeOH:H₂O [75:25:3]) After one hour the reaction was finished, the reaction mixture was filtered over a celite pad and the solvent was evaporated at reduced pressure, obtaining 26 mg of pure product **10** (99%). ${\left[α\right]}_{D}^{20} = + 64.01$ (c:1.95, CH₃OH)
¹H-NMR (400 MHz, CD₃OD) δ (ppm): 4.95 (d, 1H, H_{1M}, J₁₋₂ = 1 Hz), 4.79 (d, 1 H, H_{1M'}, J_{1'-2'} = 1.6 Hz), 4.07 (bs, 1 H, D₂), 3.90-3.81 (m, 4H, H_{2M}, H_{2M'}, H_{6B M}, H_{6B M'}), 3.80-3.74 (m, 3H, D₁, H_{7B}, H_{6A M'}), 3.74-3.54 (m, H, H_{6A M}, H_{3M}, H_{3M'}, H_{4M'}, H_{5M'}, H_{5M}, H_{4M}), 3.66 (s, 6H, OCH₃), 3.54-3.46 (m, 1 H, H_{7A}), 3.00 (dt, 1 H, D₅, J_{D5-D4}=J_{D5-D6ax}=12 Hz, J_{D5-D6eq}= 4 Hz), 2.90-2.78 (m, 1H, D₄), 2.84 (t, 2H, H₈, J₇₋₈ = 5.2 Hz), 2.16 (app d, 1H, D₆ₐₓ, J_{gem}= 13.6 Hz), 2.04 (app d, 1H, D₃ₐₓ, J_{gem}= 14.0 Hz), 1.83 (t, 1H, D_{3eq}), 1.75 (t, 1H, D_{6eq}). ¹³C-NMR (100 MHz, CD₃OD) δ (ppm): 177.25, 177.02 (COOCH₃), 101.87 (C1_{M'}), 100.37 (C1_{M}), 76.06 (C1_{D}), 75.52 (C4_{M}), 74.08 (C4_{M'}), 72.66, 72.57, 72.52 (C2_{M}, C2_{M'}, C3_{M}, C3_{M'}), 71.98 (C2_{D}), 70.36 (C6_{M'}), 70.01 (C6_{M}), 68.68, 68.59 (C5_{M}, C5_{M'}), 63.01 (C7), 52.36 (OCH₃), 42.13 (C₈), 40.39 (C4_{D}), 40.15 (C5_{D}), 28.91 (C6_{D}), 28.34 (C3_{D}). MS (ESI) : Calculated for [C₂₄H₄₂NO₁₆]⁺ = 600.25, experimental = 600.3.

### 6) Synthesis of the tetravalent dendron 12 of the α(1,2)α(1,6)pseudomannotriose

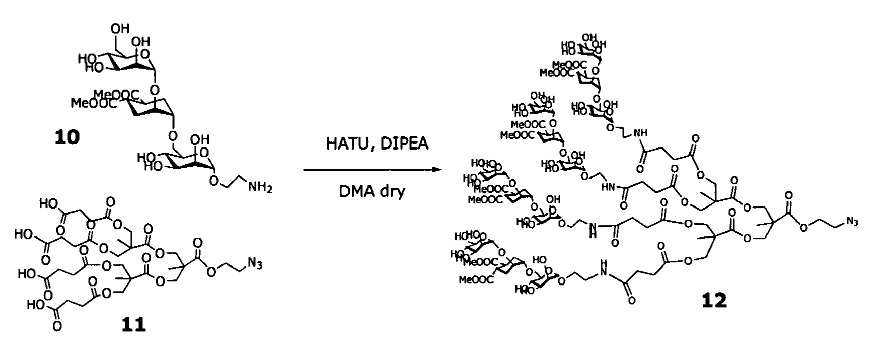

To a solution of the scaffold dendron **11** (4.5 mg, 0.00539 mmol, 1 eq) in 100 µL of dry DMA under nitrogen atmosphere, HATU (17 mg, 0.04336 mmol, 8 eq) and DIPEA (15 µL, 0.0867 mmol, 16 eq) were added. After 15 minutes a solution of **10** (26 mg, 0.04336 mmol, 8eq) in dry DMA (170 µL) was added. The reaction was stirred at room temperature for 3 days. MALDI mass analysis of a sample of the reaction mixture showed the completion of the reaction. The reaction mixture was diluted in methanol and charged directly onto a LH-20 Sephadex column in methanol. Slow elution led to the purification of the product **12** that was isolated in good yield (15.6 mg, 92% yield). ${\left[α\right]}_{D}^{20} =$ +55.20 (c: 0.735, CH₃OH). ¹H-NMR (400 MHz, D₂O) δ (ppm): 5.00 (s, 4H, H_{1M'} assigned on the basis of interglycosidic NOE signal in ROESY experiment between H_{1M'} and D₁ and D_{3eq}), 4.84 (s, 4H, H_{1M}), 4.33 (bs, 6H, OCH₂CH₂N₃, CH₂O a), 4.24 (bs, 8H, CH₂O b), 4.03 (bs, 1 H, D₂), 3.97 (bs, 4H, H_{2M'}), 3.92 (bs, 4H, H_{2M}), 3.89-3.80 (m, 12H, H_{3M'}, H_{6M'B}, H_{6M B}), 3.80-3.53 (m, 38H, D₁, H_{6M'A}, H_{6M A}, H_{3M}, H_{4M}, H_{4M'}, H₇, H_{5M}, H_{5M'}, CH₂N₃), 3.69 (s, 24H, COOCH₃), 3.41 (bs, 8H, H₈), 3.46-3.38 (m, 8H, D₄, D₅), 2.72-2.62 (m, 8H, CH₂COO), 2.60-2.49 (m, 8H, CH₂CONH), 2.24-2.06 (m, 8H, D_{6eq}, D_{3eq}), 1.92-1.72 (m, 8H, D₆ₐₓ, D₃ₐₓ), 1.32 (s, 3H, CH₃ a'), 1.25 (s, 6H, CH₃ b'). ¹³C-NMR (100 MHz, D₂O) δ (ppm): 181.9 (COO); 178.2, 177.9 (COOCH₃); 174.7, 174.66 (COOCH₂ b, CONH); 174.3 (COOCH₂ a); 100.5 (C1_{M}); 99.2 (C1_{M'}); 75.1 (C1_{D}); 71.5 (C2_{D}); 74.0, 72.5, 71.2, 71.1 (C3_{M}, C3_{M'}, C4_{M}, C4_{M'}); 71.1 (C2_{M'}); 70.6 (C2_{M}); 68.5 (C6_{M}); 67.5, 67.4 (C5_{M}, C5_{M'}); 66.8 (C7, OCH₂ a, OCH₂ b), 66.5 (OCH₂CH₂N₃), 61.6 (C6_{M'}), 53.2 (CH₃O); 50.0 (CH₂N₃); 47.3, 47.0 (Cquat a", b"); 39.6 (C4_{D}, C5_{D}); 39.5 (C8); 30.6 (CH₂CONH); 29.8 (CH₂COO); 27.6, 27.3 (C6_{D}, C3_{D}), 17.5 (CH₃ a', b').
MS (MALDI) Calculated for [C₁₂₉H₂₀₁N₇O₈₂+Na]⁺ = 3184.97, experimental = 3184.6.

### EXAMPLE 2

### Synthesis of compound 12a

### 1) Synthesis of the α(1,2)pseudomannobiose 13

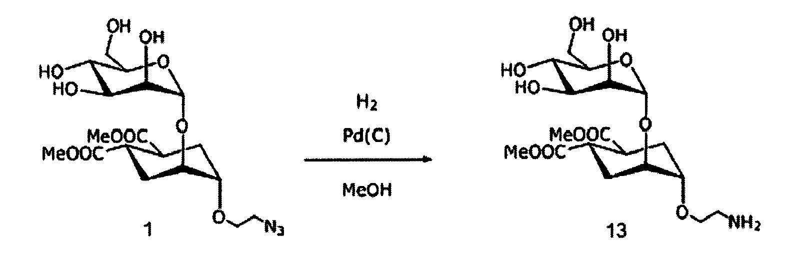

To a solution of the azide **1** (71.9 mg, 0.1551 mmol, 1eq) in MeOH (7.8 mL) a catalytic amount of Pd on carbon was added. The suspension was put under hydrogen atmosphere and stirred at room temperature monitoring by TLC ([8:2] CHCl₃:MeOH). After two hours the reaction was finished, the reaction mixture was filtered over a celite pad and the solvent was evaporated at reduced pressure, obtaining 68 mg of pure product **13** (quantitative yield). ${\left[α\right]}_{D}^{20} = + 48.3$ (c: 0.60, CH₃OH). ¹H-NMR (400 MHz, CD₃OD) 6 (ppm): 4.96 (s, 1H, H₁), 4.05 (bs, 1H, H_{D1}), 3.90-3.83 (m, 2H, H_{6B}, H₂), 3.74-3.64 (m, 4H, D₂, H_{6A}, H₃, H_{7B}), 3.68 (s, 6H, OCH₃), 3.64-3.60 (m, 1 H, H_{7A}), 3.60-3.52 (m, 2H, H₄, H₅), 2.98-2.83 (m, 4H, Hp₄ H_{D5}, H₈), 2.17-2.03 (m, 2H, D_{3eq}, D_{6eq}), 1.87-1.74 (m, 2H, D₃ₐₓ, D₆ₐₓ).
¹³C-NMR (100 MHz, CD₃OD) δ (ppm): 177.0, 176.9 (COOCH₃); 100.3 (C1_{M}); 76.0 (C1_{D}); 75.6 (C4_{M}); 72.5, 72.4 (C2_{M}, C3_{M}); 72.1 (C2_{D}); 69.8 (C7); 68.7 (C5_{M}); 63.1 (C6_{M}); 52.5, 52.4 (COOCH₃); 41.9 (C8); 40.4, 40.2 (C4_{D}, C5_{D}); 29.0 (C6_{D}); 28.2 (C3_{D}).
HR-MS (ESI) for C₁₈H₃₁NO₁₁; [M+Na]⁺ calculated: 460.17893; experimental: 460.17826.

### 2) Synthesis of the tetravalent dendron of the α(1,2)pseudomannobiose 12a

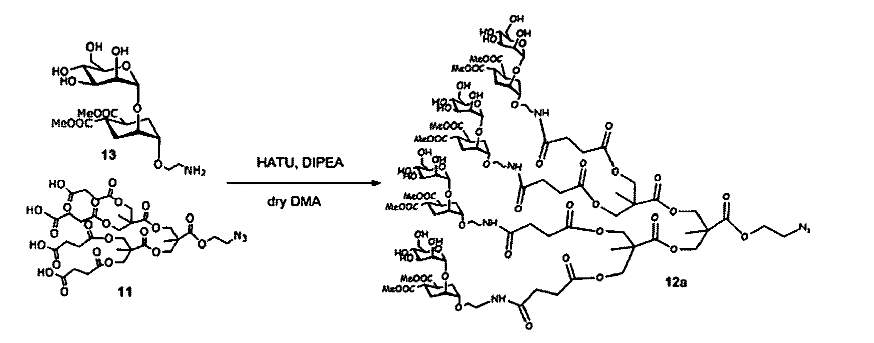

To a solution of the functionalized scaffold dendron **11** (6.0 mg, 0.00718 mmol, 1 eq) in 150 µL of dry DMA under nitrogen atmosphere, HATU (22 mg, 0.0575 mmol, 8 eq) and DIPEA (20 µL, 0.1157 mmol, 16 eq) were added. After 15 minutes a solution of **13** (23.4 mg, 0.05349 mmol, 8eq) in dry DMA (210 µL) was added. The reaction was stirred at room temperature for 3 days. MALDI mass analysis of a sample of the reaction mixture showed the completion of the reaction.
The reaction mixture was diluted in methanol and charged directly onto a LH-20 Sephadex column in methanol. Slow elution led to the purification of the product **12a,** which was isolated in good yield (16.9 mg, 94% yield). ${\left[α\right]}_{D}^{20} =$ + 36.66 (c: 0.78 , CH₃OH). ¹H-NMR (400 MHz, D₂O) δ (ppm): 4.93 (s, 4H, H₁), 4.27 (bs, 6H, OCH₂CH₂N₃, CH₂O a), 4.18 (bs, 8H, CH₂O b), 3.91 (bs, 8H, D₂, H₂), 3.78 (d, 4H, H_{6B}, J_{gem} = J_{6B-5} = 12 Hz), 3.76-3.71 (m, 4H, H₃), 3.69-3.59 (m, 8H, H_{6A}, D₁), 3.63 (s, 24H, OCH₃), 3.59-3.49 (m, 18H, H₄, H₅, H₇, CH₂N₃), 3.30 (bt, 8H, H₈), 2.91-2.72 (m, 8H, D₄, D₅), 2.65-2.56 (m, 8H, CH₂COO), 2.54-2.45 (m, 8H, CH₂CONH), 2.10-1.99 (m, 8H, D_{6eq}, D_{3eq}), 1.81-1.64 (m, 8H, D₆ₐₓ D₃ₐₓ), 1.26 (s, 3H, CH₃ a'), 1.19 (s, 6H, CH₃ b').
¹³C-NMR (100 MHz, D₂O) δ (ppm): 177.4, 177.2, 174.1, 174.0, 173.8, 173.7 (COOCH₃, COOCH₂, CONH); 98.6 (C1_{M}); 73.8 (C1_{D}); 73.5 (C4_{M}); 71.0, 70.5 (C2_{D}, C2_{M}, C3_{M}); 66.9 (C7); 66.8 (C5_{M}); 65.9 (OCH₂ a, OCH₂ b); 64.8 (OCH₂CH₂N₃); 61.0 (C6_{M}); 52.6 (CH₃O); 49.4 (CH₂N₃); 46.6, 46.4 (Cquat a", b"); 39.4 (C8); 39.0 (C4_{D}, C5_{D}); 30.1 (CH₂CONH); 29.3 (CH₂COO); 27.0, 26.7 (C6_{D}, C3_{D}), 16.9 (CH₃ a', b').
MS (ESI) for [C₁₀₅H₁₆₁N₇O₆₂]: [M+2Na]⁺⁺ calculated = 1279.7, experimental = 1279.2.
MS (MALDI, matrix DHB) [M+Na]⁺ calculated =2536.4, experimental = 2537.2.

### EXAMPLE 3

Synthesis of third generation bis(methoxyol)propanoic (bis-MPA) based dendrimer bearing α(1,2) α(1,6)pseudomannotriose **15**

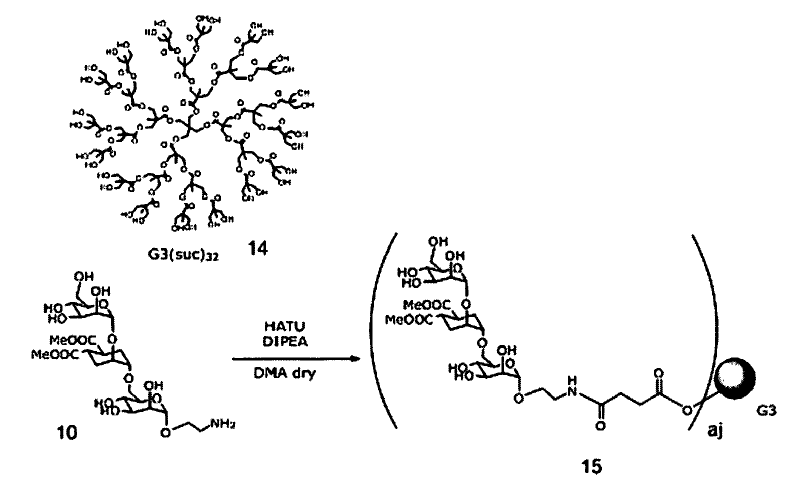

To a solution of the third generation bis(methoxyol)propanoic (bis-MPA) based dendrimer **14** (7.6 mg, 0.001153 mmol, 1 eq) in 100 µL of dry DMA under nitrogen atmosphere, HATU (28.1 mg, 0.074 mmol, 64 eq) and DIPEA (26 µL, 0.148 mmol, 128 eq) were added. After 15 minutes a solution of **10** (35.7 mg, 0.06 mmol, 52 eq) in dry DMA (230 µL) was added. The reaction was stirred at room temperature for 2 days. MALDI mass analysis of a sample of the reaction mixture showed the completion of the reaction.

The reaction mixture was diluted in methanol and charged directly onto a LH-20 Sephadex column in methanol. Slow elution led to the purification of the product 15 that was isolated in good yield (25.5 mg, 88% yield). ¹H-NMR (400 MHz, D₂O) δ (ppm): 5.00 (s, H_{1M'}), 4.84 (s, H_{1M}), 4.40-4.10 (m, OCH₂CH₂N₃, CH₂O a, CH₂O b, CH₂O c), 4.04 (s, D₂), 3.97 (bs, H_{2M'}), 3.92 (s, H_{2M}), 3.89-3.53 (m, H_{3M'}, H_{6M'B}, H_{6M B}, D₁, H_{6M'A}, H_{6M A}, H_{3M}, H_{4M}, H_{4M'}, H₇, H_{5M}, H_{5M'}, CH₂N₃), 3.69 (s, COOCH₃), 3.68 (s, COOCH₃), 3.49-3.34 (m, 8H, H₈), 3.00-2.85 (m, D₄, D₅), 2.73-2.59 (m, CH₂COO), 2.59-2.46 (m, CH₂CONH), 2.27-2.03 (m, D_{6eq}, D_{3eq}), 1.89-1.71 (m, D₆ₐₓ D₃ₐₓ), 1.40-1.15 (m, CH₃ a', CH₃ b', CH₃ c'). ¹³C-NMR (100 MHz, D₂O) δ (ppm): 177.3, 177.0, 173.5, 173.0, 98.7, 74.7, 73.5, 72.2, 71.0, 70.9, 70.7, 70.6, 70.1, 68.2, 67.1, 66.9, 66.2, 65.4, 61.1, 52.6, 46.4, 39.1, 39.0, 39.0, 30.1, 29.2, 27.2, 26.9, 17.3. MS (MALDI, matrix: SIN): distribution centered on 30 sugar loaded, for 32 sugars [M+Na]⁺ calculated: 25222.75, found: 25227.9.

### EXAMPLE 4

### Infection Studies

B-THP-1/DC-SIGN cells are derived from B-THP-1 human B cell line by transfection of DC-SIGN expression vector in order to express high levels of the DC-SIGN receptor. This cell line supports efficient DC-SIGN mediated HIV transmission and is a wide-used model system to mimic HIV capture and transmission to T-lymphocytes by dendritic cells (see, as a general reference, Geijtenbeek, T.B.H.; Torensma, R.; Van Vliet, S.J.; van Duijnhoven, G.C.F.; Adema, G.J:; van Kooyk, Y.; Figdor, C.G. Cell, 2000, 100, 575- 585). B-THP-1/DC-SIGN cells were preincubated for 30 minutes either in the presence or in the absence of the DC-SIGN inhibitors prior to 3 hours exposure to the virus (the R5 tropic laboratory-adapted strain HIV-1 BaL) in the continued presence of inhibitors. Mannan is known to inhibit DC-SIGN mediated viral infection (Geijtenbeek, T.B.H.; Torensma, R.; Van Vliet, S.J.; van Duijnhoven, G.C.F.; Adema, G.J:; van Kooyk, Y.; Figdor, C.G. Cell, 2000, 100, 575- 585; Hong, P.W.-P. et al, J. Virol., 2002, 76(24), 12855-12865; Lin G. et al J. Virol. 2003, 77, 1337-46) and was used as positive control. Non transfected B-THP-1 cells were used as a negative control in initial experiments. After washing, the B-THP-1/DC-SIGN cells were co-cultured with activated CD4+ T lymphocytes from healthy volunteer donors. Viral infection of CD4+ T lymphocytes was assessed by measuring the concentration of the HIV core protein p24 in the co-culture supernatants by ELISA. Each point was obtained in triplicate using CD4+ T lymphocytes from three different donors.

The results obtained for compound **12 (SARA 133)** are reported in **Figure 1**. In particular, at 50 µM, the compound appears to reduce by over 90% the trans-infection of CD4+ T lymphocytes; moreover, at 100 µM and at 250 µM inhibition is almost complete. **Figures 8** and **Figure 9** show the results of the same experiment performed with HIV-1 strain IIIB and HIV-1 strain 89,6 (dual tropic), respectively.

In **Figure 2** the data for HIV-1 BaL infection are compared to those obtained using the monovalent antagonist **2b**, which clearly needs a much higher concentration to reach a similar inhibition level.

Even more striking is the comparison obtained when the B-THP-1/DC-SIGN cells were first incubated with the inhibitors and then washed with buffer before adding the virus (BaL). In this case (**Figure 3**) the tetravalent system **12** showed surprising cell adhesion properties, which resulted in a still efficient antiinfective action with >94% of inhibition at 250 µM, the lowest concentration tested in this type of assay. On the contrary, the monovalent compound was unable to persist on the cell surface, even when used at 5 mM concentration, leading to no antiinfective activity in the trans-infection assay (Figure 3). Dose-response curves were obtained for **12** using CD4+ T lymphocytes from healthy donors who are either genetically resistant (P1) or genetically susceptible (P2) to HIV infection. B-THP-1/DC-SIGN cells were challenged with HIV-1 (BaL strain) in the presence of increasing concentrations of **12**. After washing and co-culture with the CD4+ T lymphocytes analysis of p24 concentration in the supernatants allowed to assess viral infection. Each point was obtained in duplicate. At 100 µM the inhibition of infection was complete for both donors and a 5 µM IC₅₀ could be estimated (**Figure 4**).

Infection studies were performed not only on laboratory adapted HIV strains but also on viral strains isolated from patients. In particular clades V6 (CCR5 tropic) (**Figure 5**) and V17 (CXCR4 tropic) (**Figure 6**) were tested: **12** showed >99% inhibition at 50 µM concentration.
7-aminoactinomicin D (7-AAD) labelling of the cells after the incubation period and cytofluorimetric analysis showed that the anti-infective properties of the inhibitors are real and not an epiphenomenon, due to cell death. Thus, the data for **12** shown in **Figure 7** indicate that the percentage of 7-AAD positive cells (apoptotic cells) did not change significantly in the absence of the compound or in its presence up to 250 µM (the highest concentration tested in the infection studies). Similar data were obtained after incubating the cells with **12** for **24** hours (not shown).

Figure 10 shows the anti-infective activity of a group of multivalent compounds. **12** and **12a** are within the present invention, **Man4** is a tetravalent presentation of mannose on the same scaffold; **G3PS-di24** is a fully succinylated BH30 dendrimer (which is a third generation dendrimer based on 2,2'-bis(methoxyol)propanoic acid (bis-MPA)) presenting 24-26 copies of the amine derived from 1 (see above); **G3Man32** is a fully succinylated BH30 dendrimer presenting 32 copies of mannose.

The infection data in Figure 10 show that mannosylation alone affords compounds which are unable to block the infection. Both the tetramannosylated dendron **Man4** and the full BH30 dendrimer presenting 32 copies of mannose **G3Man32** do not reduce the infection level below 30%, even at 100 µM concentration. The tetravalent pseudo-disaccharide **12a** is active at 100 µM.
**12** has the same anti-infective activity with respect to **G3PS-di24** at the same concentration, but its molecular weight is about 4 times lower. Advantageously, **12** is chemically defined and is easier and cheaper to be produced.

The compounds within the another embodiment of the invention wherein within the meaning of **X, M** is 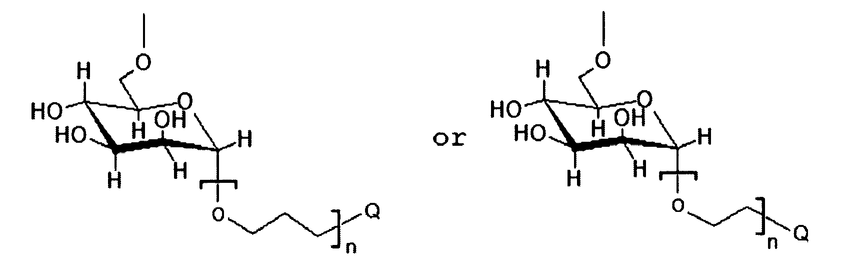 and the **Z** scaffold is a dendrimer based on 2,2'-bis(methoxyol)propanoic (bis-MPA) and, in particular,
is a first or second or higher generation bis(methoxyol)propanoic (bis-MPA) based dendrimer (see, for instance, at Fig. 12), surprisingly show to be more active than **G3PS-di24** as well.

## Claims

1. A compound of general formula (I):
**[X-Y]_{az}-Z (I)**
wherein **X** is a saccharidic or pseudosaccharidic unit of formula **M-D-M'**, wherein **M'** is of formula 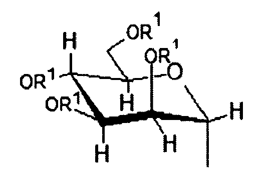 wherein R¹ is H, **D** is of formula 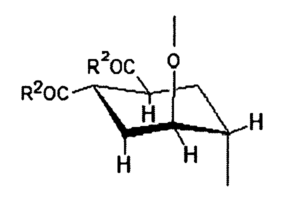 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group is a C3-C6 saturated or partially unsaturated cyclic- or heterocyclic ring or a C5-C6 aromatic or heteroaromatic ring, optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH); or R³ is an **A** group as above defined; and **M** is -(O-CH₂-CH₂)ₙ-**Q** or -(O-CH₂-CH₂-CH₂)ₙ-**Q** or 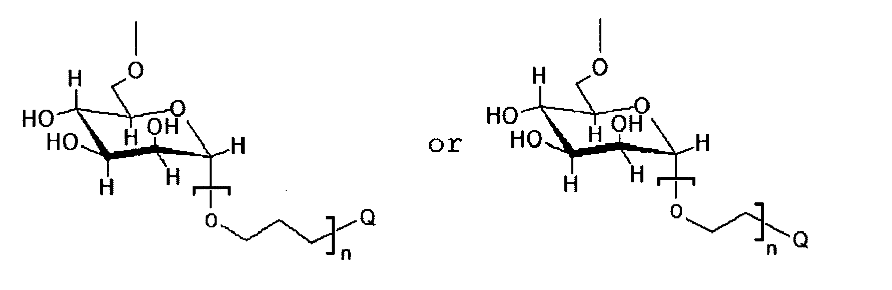 wherein **Q** is an heteroatom selected from oxygen, sulphur and nitrogen linked to the scaffold **Z** through a **Y** linker and n is 1 to 3; the **Y** linker is selected from an amino acidic residue or a succinic acid or diglycolic acid residue, **Z** is a scaffold selected from liposomes, nanoparticles, polymers, dendrimers or dendrons and **az** is the functionality of said scaffold **Z** and it is comprised between 1 and 150, provided that when in the **X** moiety R¹ is H, R² is methoxy, **M** is -(O-CH₂-CH₂)ₙ**-Q** wherein n is 1 and **Q** is nitrogen and **Y** is a succinic acid residue, the **Z** scaffold is not the dendrimer of formula

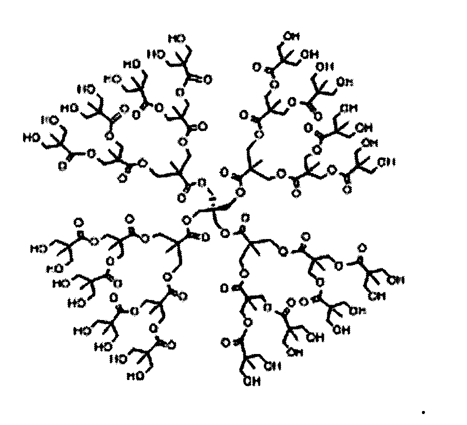

2. A compound of formula (I) according to claim 1, wherein R² is methoxy or is -NHR³, wherein R³ is an **A** group selected among cyclopropan, cyclohexil, phenyl, benzyl or benzyl meta or para substituted with methoxy (-OCH₃), nitro (-NO₂), carboxy (-COOH), methylester (-COOCH₃), methyl (-CH₃), methoxy (-OCH₃), amino (-NH₂), isopropyl (-CH(CH₃)₂) or R³ is 1,3, thiazole or methyl substituted 1,3-thiazole or R³ is a methyl group (-CH₃) substituted with any of said **A** groups.

3. A compound according to claims 1 or 2 having any one of the following **X** moieties: 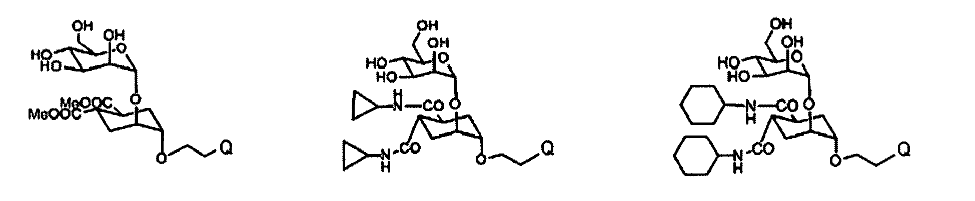 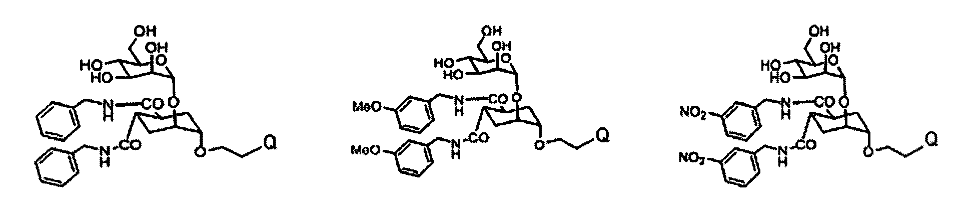 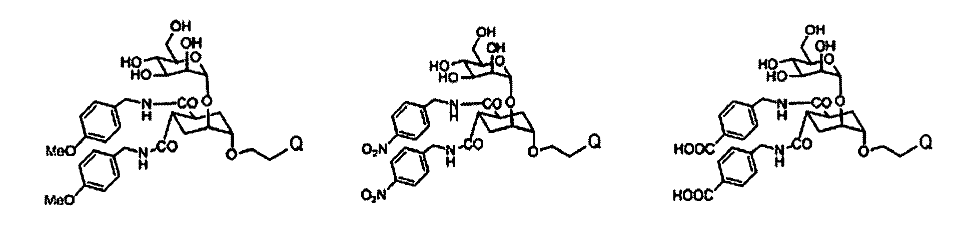 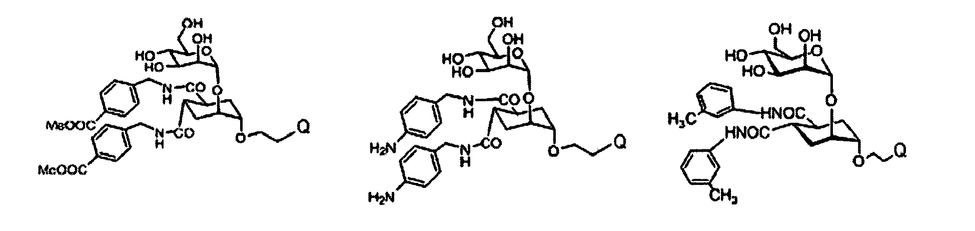 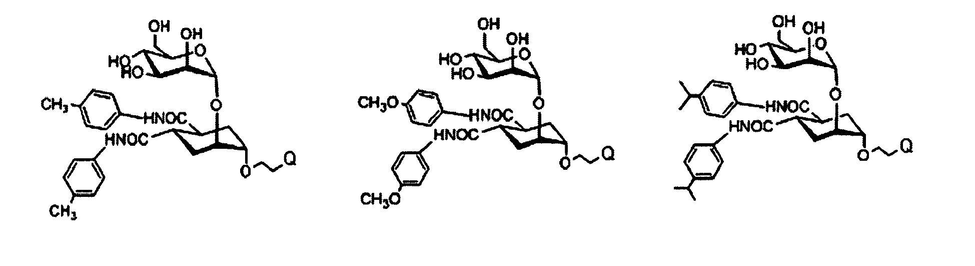 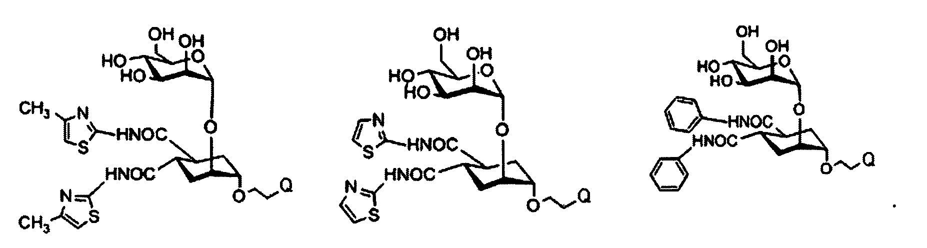 wherein **Q** is an heteroatom linked to the scaffold **Z** through the **Y** linker.

4. A compound according to any of the preceding claims, wherein the linker **Y** is a succinic acid residue.

5. A compound according to any of the preceding claims wherein the **Z** scaffold is selected from liposomes, nanoparticles, polymers, bis(methoxyol)propanoic (bis-MPA) based dendrons and first or second or higher generation bis(methoxyol)propanoic (bis-MPA) based dendrimers or PAMAM based dendrons or dendrimers.

6. A compound according to any of the preceding claims wherein the **Z** scaffold is any of the following: 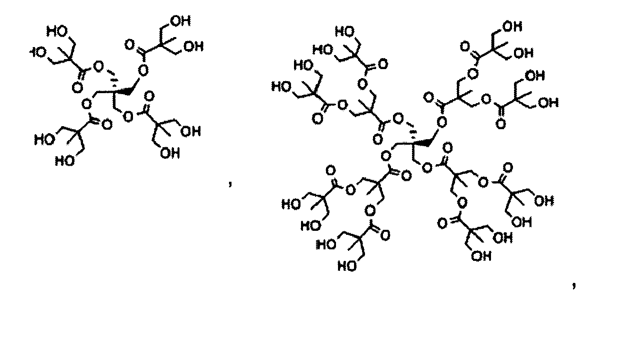 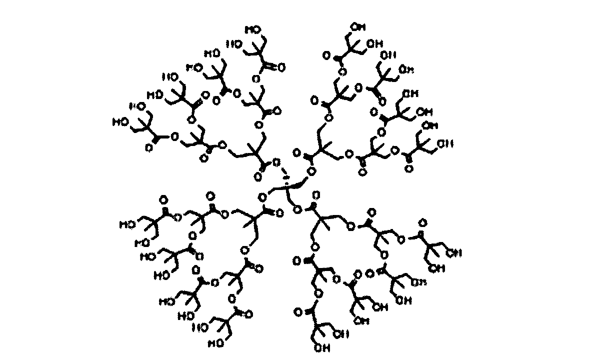 or

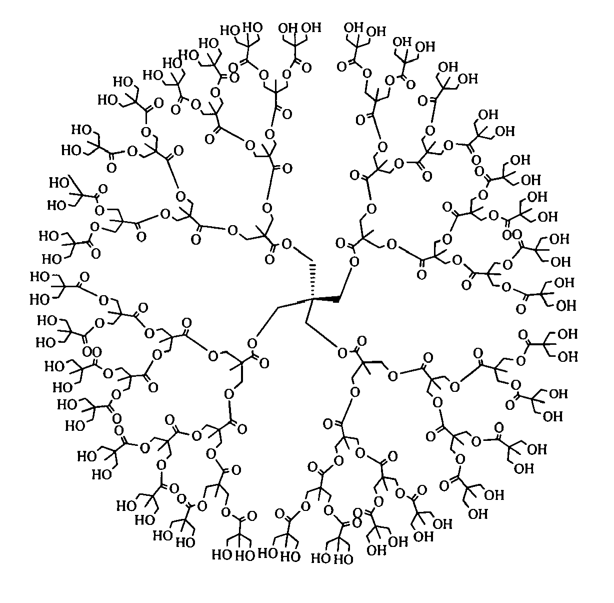

7. A compound having the general formula (Ia):
**[X-Y]_{aw}-W (Ia)**
wherein **X** is a saccharidic or pseudosaccharidic unit of formula **M-D-M',** wherein **M'** is of formula 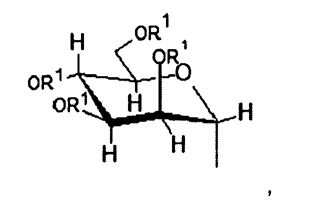 wherein R¹ is H, **D** is of formula 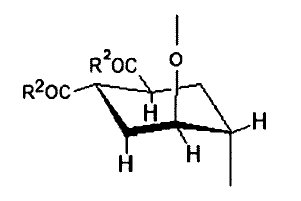 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group is a C3-C6 saturated or partially unsaturated cyclic- or heterocyclic ring or a C5-C6 aromatic or heteroaromatic ring, optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH); or R³ is an **A** group as above defined; and **M** is -(O-CH₂-CH₂)ₙ-**Q** or -(O-CH₂-CH₂-CH₂)n-**Q** or 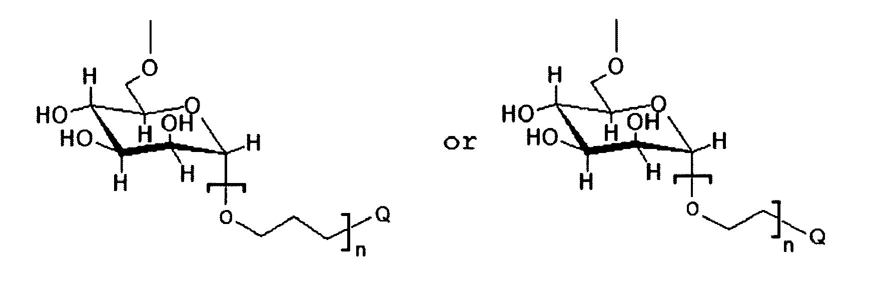 wherein **Q** is an heteroatom selected from oxygen, sulphur and nitrogen and is linked to the **W** scaffold through the **Y** linker and n is 1 to 3; the **Y** linker is selected from an amino acidic residue or a succinic acid or diglycolic acid residue, **W** is dendron based on 2,2'-bis(methoxyol)propanoic acid (bis-MPA), such as 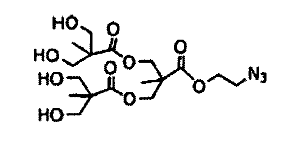 or **W** is a poly(amidoamine)(PAMAM) based dendron and **aw** is the functionality of the scaffold **W** and it is comprised between 1 and 100.

8. A compound according to any one of the preceding claims having the following formula: 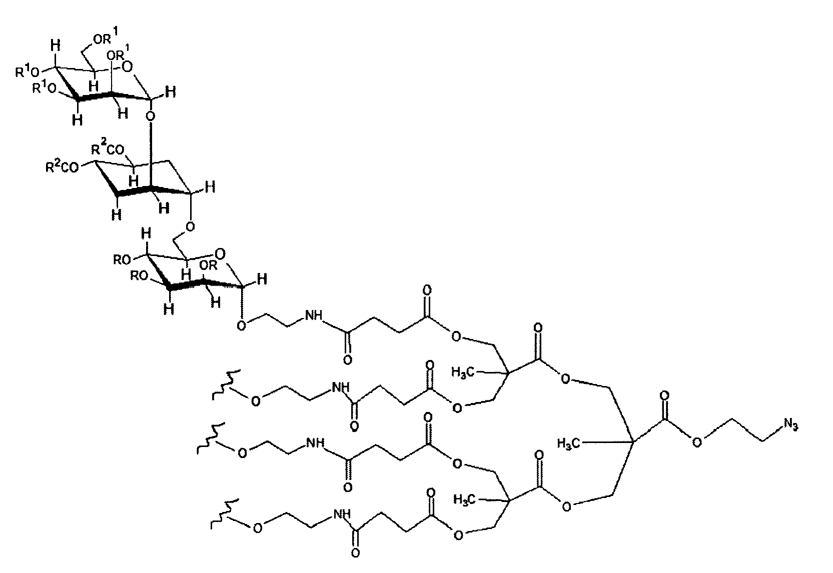 wherein R and R¹ are hydrogen and R² is methoxy.

9. A compound according to any one of the preceding claims having the following formula: 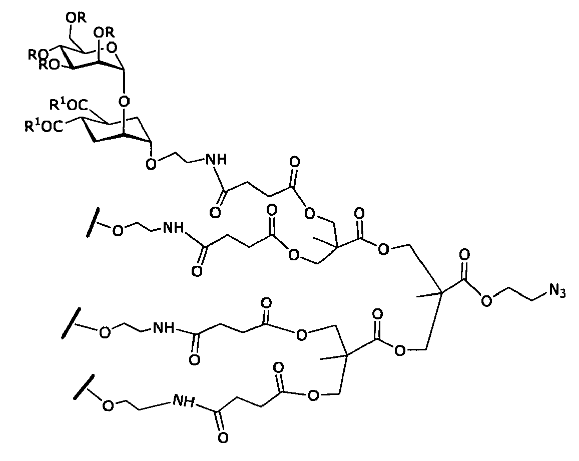 wherein R is hydrogen and R¹ is methoxy or is
-NHR³, wherein R³ is an **A** group selected in the group comprising cyclopropan, cyclohexil, phenyl, benzyl or benzyl meta or para substituted with methoxy (-OCH₃), nitro (-NO₂), carboxy (-COOH), methylester (-COOCH₃), methyle (-CH₃), methoxy (-OCH₃), amino (-NH₂), isopropyl (-CH(CH₃)₂) or R³ is 1,3, thiazole or methyl substituted 1,3-thiazole; or R³ is a methyl group (-CH₃) substituted with said **A** group.

10. A compound having the general formula (**Ib**),
**[X-Y]ₐⱼ-J (Ib)**
wherein **X** is a saccharidic or pseudosaccharidic unit of formula **M-D-M'**, wherein **M'** is of formula 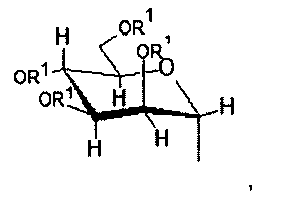 wherein R¹ is H, **D** is of formula 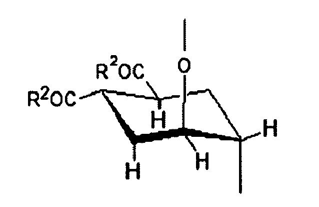 wherein R² may be hydroxyl (-OH), alkoxy (-O-R'), wherein R' is a C1-C8 linear or branched, saturated or unsaturated alkyl chain and is preferably a methoxy group (-OCH₃), or R² is -N-R³₂, wherein each R³ may be independently H, a C1-C8 linear or branched, saturated or unsaturated alkyl chain, optionally substituted with an **A** group, wherein the **A** group is a C3-C6 saturated or partially unsaturated cyclic- or heterocyclic ring or a C5-C6 aromatic or heteroaromatic ring, optionally substituted at any available position with one or more of hydroxyl (-OH), C1-C8 linear or branched, saturated or unsaturated alkyl chain, C1-C8 linear or branched, saturated or unsaturated alkoxy carbon chain, amino (-NH₂), thiol (-SH), nitro (-NO₂), carboxy (-COOH); or R³ is an **A** group as above defined; and **M** 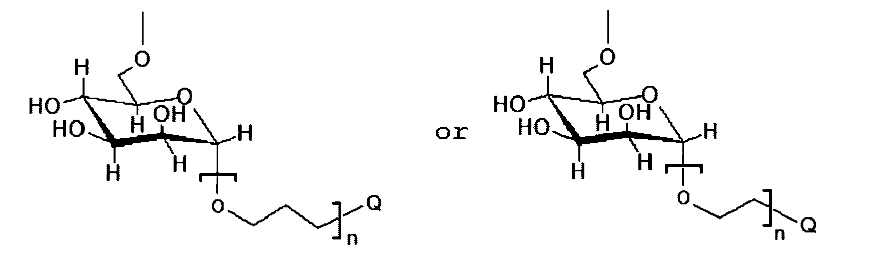 wherein **Q** is an heteroatom selected from oxygen, sulphur and nitrogen and is linked to the **J** scaffold through a **Y** linker and n is 1 to 3; or wherein R² is -N-R³₂ as above disclosed and **M** is -(O-CH₂-CH₂)ₙ-Q or -(O-CH₂-CH₂CH₂)ₙ-Q, wherein **Q, Y** and **n** are as above disclosed, wherein each R³ may be independently H, a C1-C8 linear or branched, saturated or unsaturated alkyl chain, optionally substituted with an **A** group; the **Y** linker is selected from an amino acidic residue or a succinic acid or diglycolic acid residue, J is first or second or higher generation dendrimer based on 2,2'-bis(methoxyol)propanoic acid (bis-MPA) or a poly(amidoamine) (PAMAM) based dendrimer and aj is the functionality of the scaffold **J** and it is comprised between 1 and 150.

11. A compound according to claim 10, wherein the **J** scaffold is any of the following 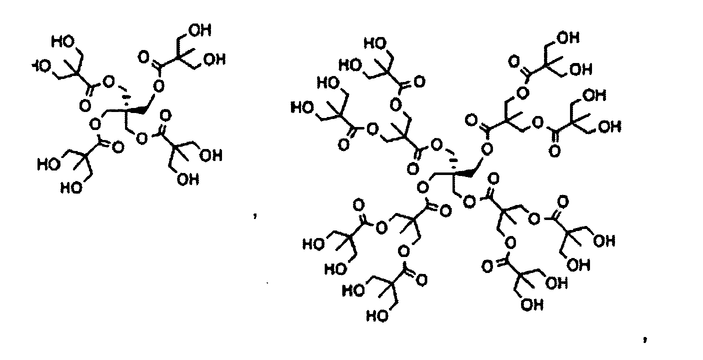 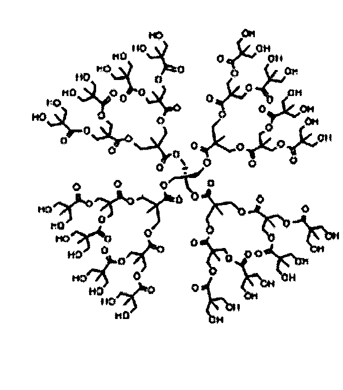 or

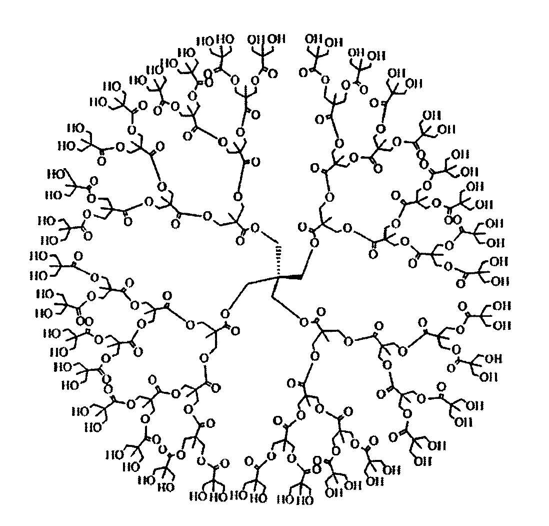

12. A compound according to claim 10 or 11 of formula 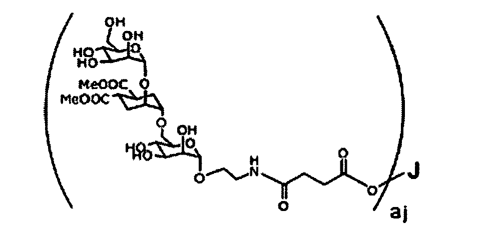 wherein **aj** is 32 and **J** is a third generation 2,2'-bis(methoxyol)propanoic acid (bis-MPA) based dendrimer.

13. Use of the compounds of formula **[X-Y]_{az}-Z (I)** as a medicament for the treatment and/or prevention of HIV, Ebola, Dengue, Hepatitis C, SARS or tuberculosis infections.

14. Use of the compounds of formula **[X-Y]_{aw}-W (Ia)** as a medicament for the treatment and/or prevention of HIV, Ebola, Dengue, Hepatitis C, SARS or tuberculosis infections.

15. Use of the compounds of formula **[X-Y]ₐⱼ-J** (**Ib**) as a medicament for the treatment and/or prevention of HIV, Ebola, Dengue, Hepatitis C, SARS or tuberculosis infections.

16. Use of the compound of formula 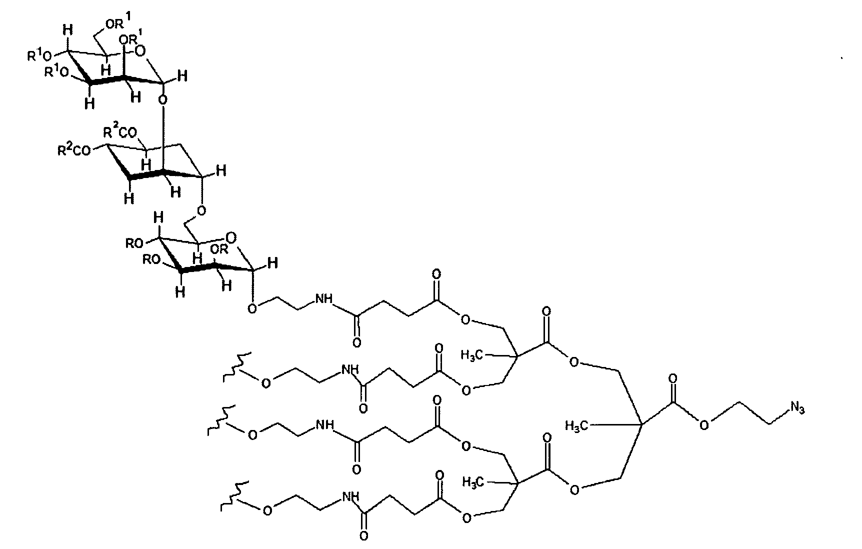 wherein R and R¹ are hydrogen and R² is methoxy; or of formula 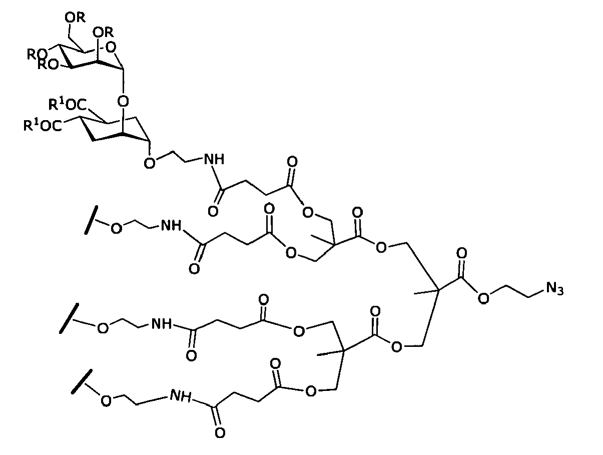 wherein R is hydrogen and R¹ is methoxy or is
-NHR³, wherein R³ is an **A** group selected in the group comprising cyclopropan, cyclohexil, phenyl, benzyl or benzyl meta or para substituted with methoxy (-OCH₃), nitro (-NO₂), carboxy (-COOH), methylester (-COOCH₃), methyle (-CH₃), methoxy (-OCH₃), amino (-NH₂), isopropyl (-CH(CH₃)₂) or R³ is 1,3, thiazole or methyl substituted 1,3-thiazole; or R³ is a methyl group (-CH₃) substituted with said **A** group, as a medicament for the treatment and/or prevention of HIV, Ebola, Dengue, Hepatitis C, SARS or tuberculosis infections.

17. Use of the compound of claim 12 as a medicament for the treatment and/or prevention of HIV, Ebola, Dengue, Hepatitis C, SARS or tuberculosis infections.

18. A pharmaceutical composition comprising a suitable effective amount of one or more of the compounds according to claims 1 to 12 together with pharmaceutically acceptable excipients.

19. The pharmaceutical composition according to claims 18, wherein said excipients and/or additives selected in the group comprising diluent, solvents, bulking agents, fillers, reological modifier, stabilizers, binders, lubricants, disintegrant, preservatives, pH adjusting agents, buffers, antioxidant, chelating agents, plasticizer, polymers, emulsifiers, edulcorants, flavoring agents, alone or in combination thereof.

20. The pharmaceutical composition of any one of claim 18 or 19 for the oral, including buccal or sublingual; rectal; nasal; transdermal; vaginal; or parenteral, including subcutaneous, intramuscular, intravenous or intradermal or topical administration.
